(19)

Europäisches
Patentamt
European
Patent Office
Office européen
des brevets

(11) **EP 2 465 437 A2**

(12) **EUROPEAN PATENT APPLICATION**

(43) Date of publication:
   **20.06.2012 Bulletin 2012/25**

(51) Int Cl.:
   *A61B 7/00* (2006.01)

(21) Application number: **11191374.5**

(22) Date of filing: **30.11.2011**

<table>
<tr><td>

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**
   Designated Extension States:
   **BA ME**

(30) Priority: **15.12.2010  JP 2010278706**

(71) Applicant: **Sony Corporation
   Tokyo 108-0075 (JP)**

</td><td>

(72) Inventors:
   • **Abe, Mototsugu**
     **Minato-ku, Tokyo 108-0075 (JP)**
   • **Myoga, Chika**
     **Minato-ku, Tokyo 108-0075 (JP)**
   • **Nishiguchi, Masayuki**
     **Minato-ku, Tokyo 108-0075 (JP)**

(74) Representative: **Jackson, Jonathan Andrew
   D Young & Co LLP
   120 Holborn
   London EC1N 2DY (GB)**

</td></tr>
</table>

(54)   **Respiratory signal processing apparatus, respiratory signal processing method, and program**

(57)   A respiratory signal processing apparatus includes a pulse-based component detection unit configured to detect a pulse-based component from a first signal acquired from a living being, and a pulse-based component removal unit configured to remove the detected pulse-based component from a second signal acquired from the living being, the second signal including respiratory sounds.

FIG. 1

EP 2 465 437 A2

**Description**

The present disclosure relates to a respiratory signal processing apparatus. In embodiments, the present disclosure relates to a respiratory signal processing apparatus and a respiratory signal processing method that remove a specific signal from a time-series respiratory signal including the respiratory sounds of a living being such as a person, and a program which enables a computer to execute the method.

It has been well-established that respiratory sounds are picked up using a stethoscope or the like to check biological conditions. It is also useful in medical situations to continuously monitor respiratory sounds. However, because noise other than respiratory sounds may often exist when respiratory sounds are monitored, it is necessary to appropriately remove such noise. The noise may include internal sounds, such as heart sounds, of the subject or patient and external ambient sounds such as speech, music, and the operation sounds or alarm sounds made by other medical instruments.

To remove the above noise, a method has been adopted in which noise components are removed using a filter. For example, a respiration rate monitoring device has been proposed which removes the components corresponding to heart sounds and other signal components through filtering using a band-pass filter having a passband of 300 Hz to 600 Hz (see, for example, Japanese Patent No. 2628690).

In actuality, however, heart sounds also include components in a frequency band as high as that of respiratory sounds, and the components may often be larger than those in the respiratory sounds. Only a filter does not necessarily provide sufficient reduction effect, and may also reduce the signal necessary for detecting respiratory sounds. Additionally, heart sounds greatly differ from person to person, and the frequency, amplitude, and phase thereof are constantly changing. Therefore, it is difficult to provide sufficient performance using a filter having a fixed coefficient alone.

Therefore, it is desirable to remove noise from a time-series respiratory signal including the respiratory sounds of a living being by using information unique to biological signals.

In a first embodiment of the present disclosure, there are provided a respiratory signal processing apparatus, a respiratory signal processing method and a program. The respiratory signal processing apparatus includes a pulse-based component detection unit configured to detect a pulse-based component from a first signal acquired from a living being, and a pulse-based component removal unit configured to remove the detected pulse-based component from a second signal acquired from the living being, the second signal including respiratory sounds. Therefore, a pulse-based component can be removed from a signal including respiratory sounds, by using information unique to a biological signal.

In the first embodiment, the first signal and the second signal may be measured respiratory signals obtained by measuring same respiratory sounds, and the pulse-based component detection unit may include the following elements. A candidate pulse-based component detection unit is configured to detect, as a candidate pulse-based component period, a period including a local maximum value in the first signal. A score calculation unit is configured to calculate a likelihood-of-pulse score for the candidate pulse-based component period. A characteristic-of-pulse determination unit is configured to determine that a signal included in a detection period is the pulse-based component, the detection period being a candidate pulse-based component period for which the likelihood-of-pulse score satisfies a certain threshold. Therefore, a pulse-based component can be detected on the basis of the likelihood-of-pulse score. In this case, the pulse-based component removal unit may include the following elements. A signal reduction unit is configured to reduce the detected pulse-based component in the first signal. A waveform synthesis unit is configured to generate a composite waveform from a signal near the detection period. A waveform addition unit is configured to add the composite waveform to the first signal. Therefore, a pulse-based component can be removed from a measured respiratory signal without causing a person to experience uncomfortable feelings.

In the first embodiment, furthermore, the first signal may be a measured heartbeat signal obtained by measuring a heartbeat waveform of the living being, and the second signal may be a measured respiratory signal obtained by measuring respiratory sounds of the living being. The respiratory signal processing apparatus may further include a time synchronization unit configured to synchronize the measured respiratory signal and the measured heartbeat signal with each other. The pulse-based component detection unit may detect the pulse-based component by estimating heart sounds from the measured heartbeat signal. The pulse-based component removal unit may remove the pulse-based component by removing the estimated heart sounds from the synchronized measured respiratory signal. Therefore, the heart sound estimated from the measured heartbeat signal can be removed from the measured respiratory signal. In this case, the pulse-based component detection unit may be a finite impulse response filter. The respiratory signal processing apparatus may further include the following elements. A breathing period detection unit is configured to detect a breathing period on the basis of a measured respiratory signal in which the heart sounds have been removed. A heartbeat period detection unit is configured to detect a heartbeat period on the basis of the measured heartbeat signal. A coefficient update unit is configured to update a filter coefficient of the finite impulse response filter during a period that is not the breathing period but is the heartbeat period. Therefore, the coefficient of a finite impulse response filter that estimates heart sounds can be updated in accordance with the respiratory conditions.

In the first embodiment, furthermore, the respiratory signal processing apparatus may further include the following elements. A time-to-frequency conversion unit is configured to convert the second signal in which the pulse-based component has been removed to generate a frequency spectrum signal for each time segment. A tone-based component detection unit is configured to detect a tone-based component from the frequency spectrum signal. A tone-based component removal unit is configured to remove the detected tone-based component from the frequency spectrum signal. A frequency-to-time conversion unit is configured to inversely convert the frequency spectrum signal in which the tone-based component has been removed. Therefore, a tone-based component can be removed from a signal including respiratory sounds, by using information unique to a biological signal. In this case, the tone-based component detection unit may include the following elements. A spectral peak detection unit is configured to detect a peak in the frequency spectrum signal for each time segment. A spectral peak tracking unit is configured to track over time segments a peak in the frequency spectrum signal for each time segment. A characteristic-of-tone determination unit is configured to determine whether or not the tone-based component exists in accordance with a peak tracked by the spectral peak tracking unit. Therefore, a tone-based component can be detected on the basis of a peak in the frequency spectrum signal for each time segment.

According to an embodiment of the present disclosure, the effect of effectively removing noise from a time-series respiratory signal including the respiratory sounds of a living being by using information unique to biological signals can be achieved.

Various respective aspects and features of the invention are defined in the appended claims. Combinations of features from the dependent claims may be combined with features of the independent claims as appropriate and not merely as explicitly set out in the claims.

Embodiments of the invention will now be described with reference to the accompanying drawings, throughout which like parts are referred to by like references, and in which:

Fig. 1 is a diagram illustrating an example configuration of a respiratory condition display system according to a first embodiment of the present disclosure;

Figs. 2A and 2B are diagrams illustrating an example of the waveform and frequency spectrum of a respiratory signal which may be used in an embodiment of the present disclosure;

Figs. 3A and 3B are diagrams illustrating an example configuration of a respiratory sound measuring device according to the embodiment of the present disclosure;

Fig. 4 is a diagram illustrating an example of the respiratory sound measuring device which is brought into close contact with the surface of the living being according to an embodiment of the present disclosure;

Figs. 5A and 5B are diagrams illustrating another example configuration of the respiratory sound measuring device according to the embodiment of the present disclosure;

Fig. 6 is a diagram illustrating an example configuration of a noise reducer according to the first embodiment of the present disclosure;

Figs. 7A to 7E are diagrams illustrating an example of the detection of a candidate pulse-based component period, which is performed by a candidate pulse-based component detection unit, and the calculation of a likelihood-of-pulse score, which is performed by a characteristic-of-pulse score calculation unit, according to the first embodiment of the present disclosure;

Figs. 8A to 8E are diagrams illustrating an example of a heart sound waveform for generating feature values according to the first embodiment of the present disclosure;

Figs. 9A to 9G are diagrams illustrating an example of a pulse-based component removal process according to the first embodiment of the present disclosure;

Fig. 10 is a diagram illustrating an example of a time-frequency signal obtained through time-to-frequency conversion according to an embodiment of the present disclosure;

Fig. 11 is a schematic diagram of an example of a time-frequency signal obtained through time-to-frequency conversion according to an embodiment of the present disclosure;

Figs. 12A to 12E are diagrams illustrating an example of a peak tracking process performed by a spectral peak detection unit according to the first embodiment of the present disclosure;

Fig. 13 is a diagram illustrating an example of frequency characteristics implemented by a tone-based component removal unit according to the first embodiment of the present disclosure;

Fig. 14 is a flowchart illustrating an example of a flow of the overall process of the noise reducer according to the first embodiment of the present disclosure;

Fig. 15 is a flowchart illustrating an example of a flow of a pulse-based component detection process according to the first embodiment of the present disclosure;

Fig. 16 is a flowchart illustrating an example of a flow of a pulse-based component removal process according to the first embodiment of the present disclosure;

Fig. 17 is a flowchart illustrating an example of a flow of a tone-based component detection process according to the first embodiment of the present disclosure;

Fig. 18 is a diagram illustrating an example configuration of a respiratory condition display system according to a second embodiment of the present disclosure;

Fig. 19 is a diagram illustrating an example configuration of a noise reducer according to the second embodiment of the present disclosure;

Fig. 20 is a diagram illustrating an overview of the process of the noise reducer according to the second embodiment of the present disclosure;

Fig. 21 is a diagram illustrating an example configuration of a time synchronizer according to the second embodiment of the present disclosure;

Fig. 22 is a diagram illustrating an example configuration of a time synchronization buffer according to the second embodiment of the present disclosure;

Fig. 23 is a diagram illustrating an example configuration of a heartbeat period detector according to the second embodiment of the present disclosure;

Fig. 24 is a diagram illustrating an example configuration of a breathing period detector according to the second embodiment of the present disclosure;

Fig. 25 is a flowchart illustrating an example of a flow of the overall process of the noise reducer according to the second embodiment of the present disclosure; and

Fig. 26 is a flowchart illustrating an example of a flow of a filter update process according to the second embodiment of the present disclosure.

Embodiments of the present disclosure (hereinafter referred to as "embodiments") will be described hereinafter. The description will be given in the following order:

1. First embodiment (an example of extracting and removing the pulse-based or tone-based component from respiratory sounds)

2. Second embodiment (an example of estimating heart sounds from a heartbeat waveform and removing the heart sounds from respiratory sounds)

1. First Embodiment

Configuration of Respiratory Condition Display System

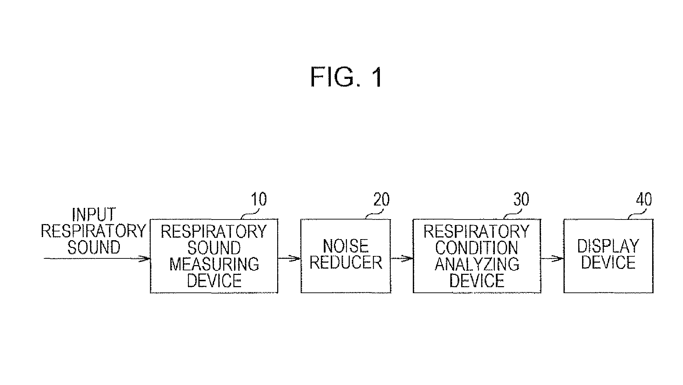

Fig. 1 is a diagram illustrating an example configuration of a respiratory condition display system according to a first embodiment of the present disclosure. The respiratory condition display system includes a respiratory sound measuring device 10, a noise reducer 20, a respiratory condition analyzing device 30, and a display device 40.

The respiratory sound measuring device 10 is configured to measure a time-series respiratory signal including the respiratory sounds of a living being such as a person. The respiratory sound measuring device 10 may be implemented as a stethoscope, by way of example, or any other tool such as a microphone positioned near the throat or a pressure sensor or an acceleration sensor touching the skin of the throat or chest may be used to measure a time-series respiratory signal. A preferred example of the respiratory sound measuring device 10 will be described below.

The noise reducer 20 is configured to perform a noise reduction process on the respiratory signal measured by the respiratory sound measuring device 10. The noise may include the ambient sounds around the living being, and the heart sounds of the living being. A preferred example of the noise reducer 20 will be described below.

The respiratory condition analyzing device 30 is configured to analyze respiratory conditions from the respiratory sounds included in the respiratory signal transmitted through the noise reducer 20. The respiratory conditions, as used herein, may include the absence or presence of a respiratory abnormality indicating whether the living being is undergoing a normal or abnormal breathing pattern, and the number of breaths per unit time.

The display device 40 is a monitor that displays the respiratory conditions analyzed by the respiratory condition analyzing device 30. Examples of the display device 40 include a liquid crystal display (LCD).

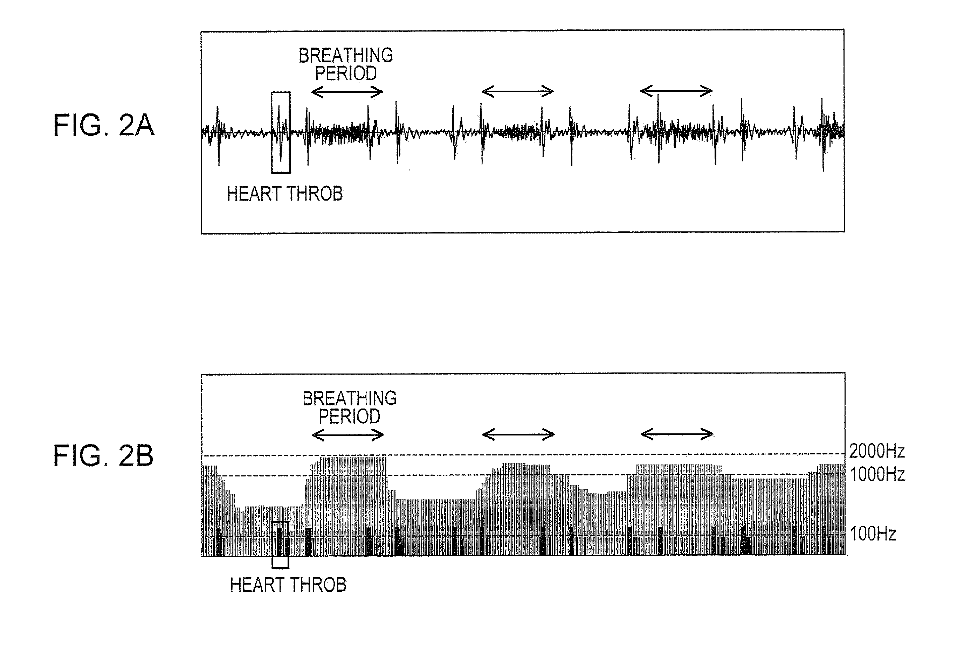

Figs. 2A and 2B are diagrams illustrating an example of the waveform and frequency spectrum of a respiratory signal which may be used in an embodiment of the present disclosure. Fig. 2A illustrates an example of the waveform of a respiratory signal, in which the horizontal axis represents time and the vertical axis represents intensity of the signal. The respiratory signal is assumed to be that measured from a living being, and includes such noise as described above other than respiratory sounds. A pulse-like waveform portion indicates a heart throb.

Fig. 2B illustrates an example of the frequency spectrum of a respiratory signal, in which the horizontal axis represents time and the vertical axis represents frequency components. As can be seen from the frequency spectrum, strong frequency components appear around 100 Hz at the timing of heart throbbing. It can also be seen that strong frequency components appear around 2 KHz at the timing corresponding to each breathing period. An example application of the above frequency spectrum analysis will be described below.

Configuration of Respiratory Sound Measuring Device

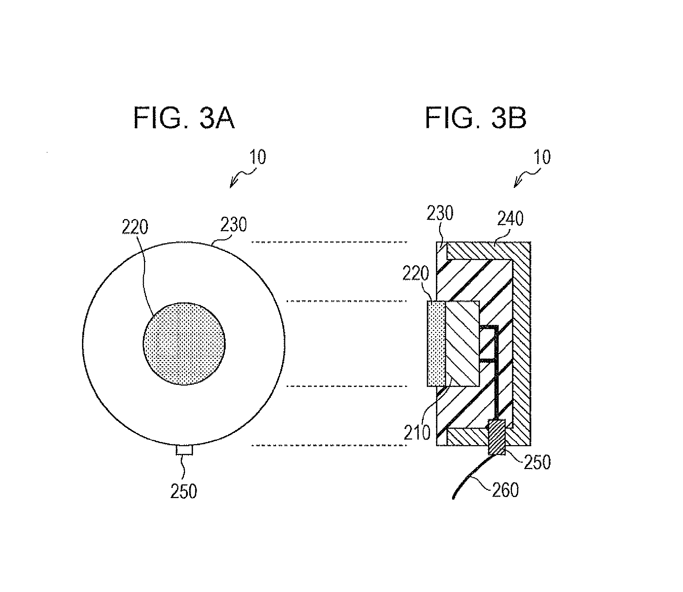

Figs. 3A and 3B are diagrams illustrating an example configuration of a respiratory sound measuring device 10 according to an embodiment of the present disclosure. Fig. 3A is an exemplary external view of the respiratory sound measuring device 10 when viewed from the front surface thereof which is in contact with the living being. Fig. 3B is a cross-sectional view of a side surface of the respiratory sound measuring device 10. The respiratory sound measuring device 10 includes a microphone 210, a sponge film 220, a soft elastic member 230, and a hard case 240. In terms of close contact with the skin, the respiratory sound measuring device 10 preferably has a diameter of about several millimeters to about several tens of millimeters.

The microphone 210 may be implemented as a general microphone such as a capacitor microphone or a microelectromechanical systems (MEMS) microphone. An output signal line extending from the microphone 210 is connected to an external line 260 via a connector 250 provided in the hard case 240.

The sponge film 220 is configured to cover a sound pickup surface of the microphone 210, which is directed toward the living being. The sponge film 220 is assumed to have a thickness of about several millimeters or more. The soft elastic member 230 is a cylindrical member formed so as to surround the microphone 210 and the sponge film 220. The soft elastic member 230 may be made of a material such as rubber. The sponge film 220 and the soft elastic member 230 deform in accordance with the shape of the skin surface to fit the body of the living being.

The hard case 240 is a cylindrical case with a lid which is configured to cover the outer surface of the soft elastic member 230. The hard case 240 maintains the shape of the respiratory sound measuring device 10, and has a sound insulation effect.

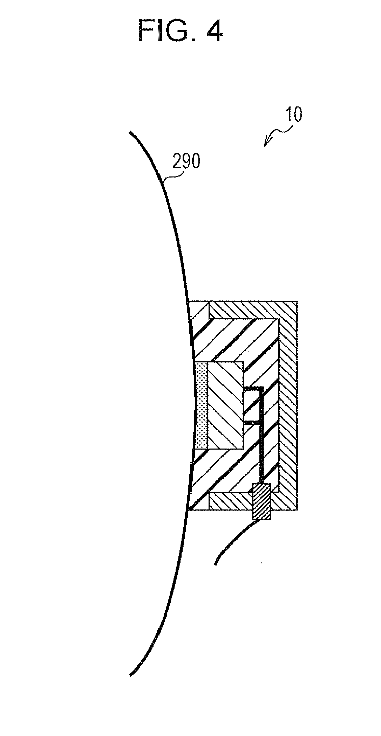

Fig. 4 is a diagram illustrating an example of the respiratory sound measuring device 10 according to an embodiment of the present disclosure, which is brought into close contact with the surface of the living being. To measure respiratory sounds, it is desirable that the respiratory sound measuring device 10 be brought into close contact with the skin surface 290 of the throat, chest, or the like. The sponge film 220 and the soft elastic member 230 are easy to deform, and thus fit the contours of the skin. Since the sponge film 220 is not infinitely compressed even when brought into strong contact with the skin surface 290, the sound pickup surface of the microphone 210 is not brought into direct contact with the skin to avoid the risk of acoustic waves being not picked up. Additionally, the sponge film 220 contains air and therefore allows acoustic waves to easily pass therethrough, whereas the soft elastic member 230 has excellent sound insulation characteristics against, in particular, high frequency components. Thus, external noise can be sufficiently reduced to a level lower than respiratory sounds.

With the above structure, furthermore, it is easy to process the top surface of the respiratory sound measuring device 10 to make it evenly flat so that the evenly processed top surface of the respiratory sound measuring device 10 can be easily fixed to the skin using, for example, a medical adhesive tape (surgical tape) or the like.

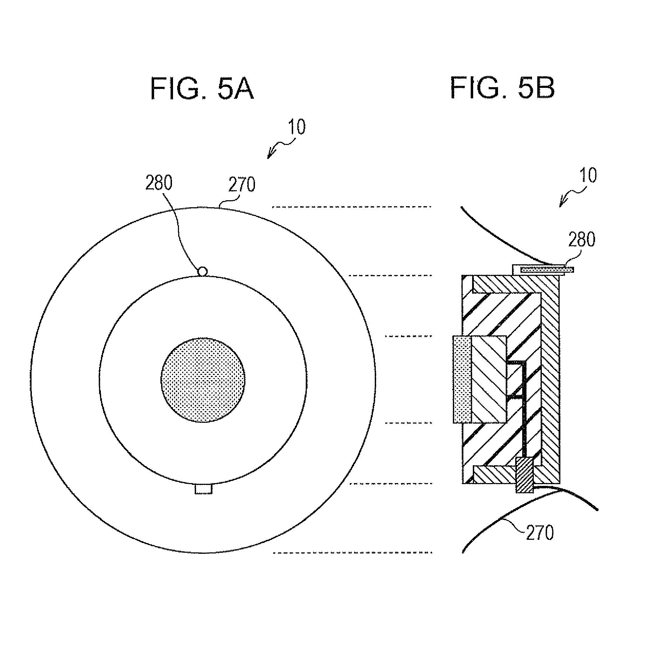

Figs. 5A and 5B are diagrams illustrating another example configuration of the respiratory sound measuring device 10 according to the embodiment of the present disclosure. Fig. 5A is an exemplary external view of the respiratory sound measuring device 10 as viewed from the front surface thereof which is in contact with the living being. Fig. 5B is a cross-sectional view of a side surface of the respiratory sound measuring device 10. In this example configuration of the respiratory sound measuring device 10, a film 270 of a soft material such as vinyl is attached to the outer side of the structure illustrated in Figs. 3A and 3B to prevent air from leaking. A decompression piston 280 is further provided so as to penetrate both sides of the film 270.

When the respiratory sound measuring device 10 is brought into close contact with the skin, the pressure between the skin and the respiratory sound measuring device 10 is slightly reduced using the decompression piston 280. Thus, air pressure is applied so that the respiratory sound measuring device 10 and the skin can come into close contact with each other. Therefore, the respiratory sound measuring device 10 can ensure a sufficiently close contact even in a case where using a surgical tape or the like is not likely to result in sufficiently close contact with the skin, such as when the living being is moving.

Configuration of Noise Reducer

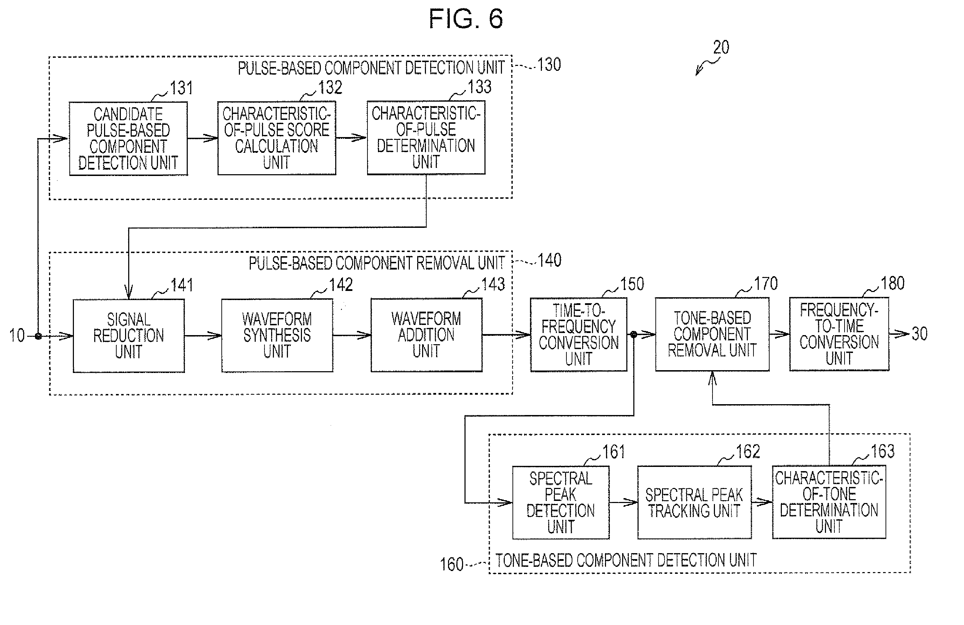

Fig. 6 is a diagram illustrating an example configuration of the noise reducer 20 according to the first embodiment of the present disclosure. The noise reducer 20 according to the first embodiment includes a pulse-based component detection unit 130, a pulse-based component removal unit 140, a time-to-frequency conversion unit 150, a tone-based component detection unit 160, a tone-based component removal unit 170, and a frequency-to-time conversion unit 180.

The pulse-based component detection unit 130 is configured to detect a pulse-based component from a measured respiratory signal measured by the respiratory sound measuring device 10. The term "pulse-based component", as used herein, refers to a signal component having a local maximum value significantly larger than other signal components, and may be caused mainly by heart sounds or noise pollution caused by other therapy devices. The pulse-based component detection unit 130 calculates a likelihood-of-pulse score, which will be described below, for a period having a local maximum value in the measured respiratory signal to determine whether or not the period is to be treated as a pulse-based component.

The pulse-based component removal unit 140 is configured to remove the pulse-based component detected by the pulse-based component detection unit 130 from the measured respiratory signal. As described below, the pulse-based component removal unit 140 reduces the detected pulse-based component and adds a composite waveform generated from near signals to remove the pulse-based component.

The time-to-frequency conversion unit 150 is configured to perform time-to-frequency conversion on the respiratory signal in which the pulse-based component has been removed by the pulse-based component removal unit 140. As described below, the time-to-frequency conversion unit 150 converts the respiratory signal into the time-frequency spectrum using, for example, a short-time Fourier transform.

The tone-based component detection unit 160 is configured to detect a tone-based component from the time-frequency spectrum obtained by the time-to-frequency conversion unit 150 through conversion. The term "tone-based component", as used herein, means a signal component that can be regarded as having periodicity in a short time, such as a vowel in speech or a musical tone. As described below, the tone-based component detection unit 160 tracks a peak value in the time-frequency spectrum for each time segment to determine whether or not the subject component is to be treated as a tone-based component.

The tone-based component removal unit 170 is configured to remove the tone-based component detected by the tone-based component detection unit 160 from the time-frequency spectrum. As described below, the tone-based component removal unit 170 is implemented as a specific-frequency component removal filter that cuts the detected tone-based component.

The frequency-to-time conversion unit 180 is configured to perform frequency-to-time conversion on the time-frequency spectrum in which the tone-based component has been removed by the tone-based component removal unit 170. As described below, the frequency-to-time conversion unit 180 performs frequency-to-time conversion using, for example, an inverse short-time Fourier transform.

Functions of Pulse-Based Component Detection Unit

The pulse-based component detection unit 130 includes a candidate pulse-based component detection unit 131, a characteristic-of-pulse score calculation unit 132, and a characteristic-of-pulse determination unit 133. The candidate pulse-based component detection unit 131 is configured to detect, as a candidate pulse-based component period, a period including a local maximum value in a measured respiratory signal measured by the respiratory sound measuring device 10. The characteristic-of-pulse score calculation unit 132 is configured to calculate the "likelihood-of-pulse score" for the candidate pulse-based component period detected by the candidate pulse-based component detection unit 131. The characteristic-of-pulse score calculation unit 132 is an example of a score calculation unit in the appended claims. The characteristic-of-pulse determination unit 133 is configured to determine, as a pulse-based component, a signal included in a detection period, where the detection period is assumed to be a candidate pulse-based component period for which the likelihood-of-pulse score calculated by the characteristic-of-pulse score calculation unit 132 satisfies a certain threshold.

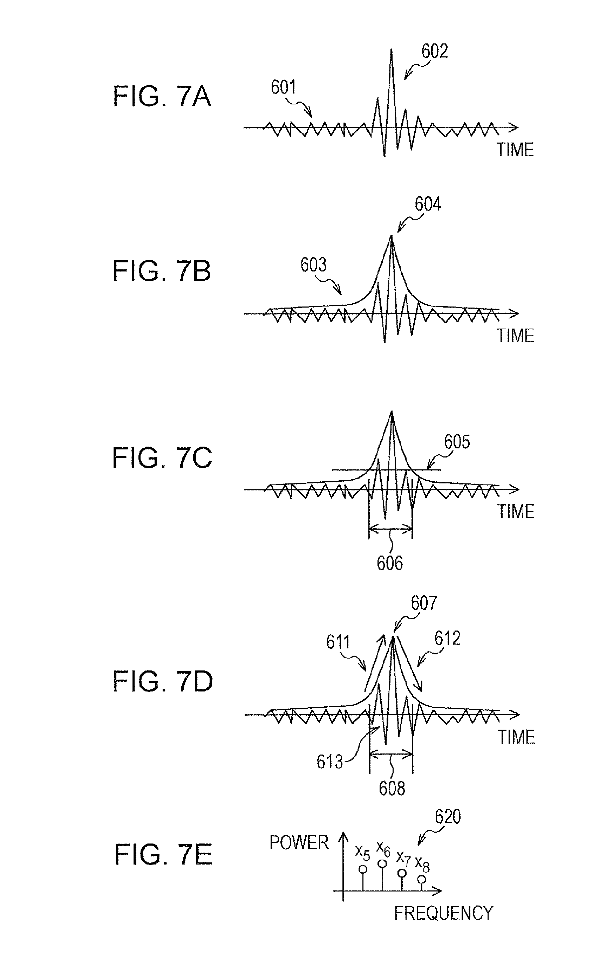

Figs. 7A to 7E are diagrams illustrating an example of the detection of a candidate pulse-based component period, which is performed by the candidate pulse-based component detection unit 131, and the calculation of a likelihood-of-pulse score, which is performed by the characteristic-of-pulse score calculation unit 132, according to the first embodiment of the present disclosure. Fig. 7A illustrates an example of a measured respiratory signal. The illustrated measured respiratory signal includes a respiratory sound waveform 601 and a pulse-based component 602.

The candidate pulse-based component detection unit 131 calculates an amplitude 603 of a signal for each period of, for example, about several tens of milliseconds, and detects, as illustrated in Fig. 7B, a position where the amplitude 603 exhibits a sufficiently large local maximum value 604. Then, as illustrated in Fig. 7C, the candidate pulse-based component detection unit 131 detects, as a candidate pulse-based component period 606, a period near the local maximum value 604 in which calculated values exceed a certain threshold 605.

The characteristic-of-pulse score calculation unit 132 calculates, from the signal in the candidate pulse-based component period 606, a "likelihood-of-pulse score" indicating whether or not the included signal component is pulse-based noise to be removed. For example, as illustrated in Fig. 7D, the characteristic-of-pulse score calculation unit 132 calculates, as feature values, a maximum amplitude value 607 ($x_0$) in the candidate pulse-based component period 606, a period length 608 ($x_1$), rates of change 611 ($x_2$) and 612 ($x_3$) of the amplitude on both sides with respect to the maximum value, and the number of zero crossings 613 ($x_4$). Also, as illustrated in Fig. 7E, the characteristic-of-pulse score calculation unit 132 calculates, as feature values, coefficients ($x_5$, $x_6$, $x_7$, $x_8$) of a short-time power spectrum 620 of the candidate pulse-based component period 606. In the illustrated example, the above feature values are nine-dimensional feature vectors $x = [x_0, x_1, ..., x_8]$.

The feature values in the feature vectors x are integrated to calculate a likelihood-of-pulse score S. The likelihood-of-pulse score S is calculated by, for example, taking a weighted sum using predetermined weighting factors w = $[w_0, w_1, ..., w_8]$ according to the following formula:

$$S = \sum_{n=0}^{N-1} w_n x_n$$

(1)

A conceivable heuristic method for determining a weighting factor w is to process multiple data sets while changing the weighting factor w and empirically determine a weighting factor w so that an appropriate result can be obtained. A conceivable learning-based method is to assign a "pulse-based component" label or an "other components" label to each of collected sample data sets and learn a weighting factor w so as to increase the scores of the "pulse-based component" group and reduce the scores of the "other components" group. The steepest descent method is used as a learning method, by way of example. First, the number of a sample data set is represented by k and the label assigned to the sample data set is represented by $z_k$. The sample data set is set to "1" if the sample data set belongs to the "pulse-based component" group and is set to "-1" if the sample data set belongs to the "other components" group. Further, if the score obtained from the sample data set is represented by $S_k$, a weighting factor w is determined using the steepest descent method so that an evaluation function J(w) represented by the formula below can be minimized using a sigmoid function Sigm(x):

$$J(w) = \sum_k (z_k - \text{Sigm}(S_k))^2,$$

where the sum $\Sigma$ ranges over all the sample data sets.

Then, the characteristic-of-pulse determination unit 133 determines whether or not the signal component in the candidate pulse-based component period is pulse-based noise using a certain threshold T as follows:

$S \geq T$; pulse-based noise
$S < T$; non-pulse-based noise.

A candidate pulse-based component period including pulse-based noise is a detection period in which the pulse-based component is detected.

In the foregoing example, the likelihood-of-pulse score S is calculated using a linear weighted sum but may also be calculated using a non-linear function. For example, a non-linear function, such as exponent, logarithm, square root, or square, of $x_n$ is represented by $x'_n$, and a weighted sum, given by Formula (1) above, is calculated for $x'_n$. In this case, the non-linear function to be used may be a function empirically determined by, as in the above case, collecting multiple sample data sets and assigning a label to each sample data set so that the assigned labels can be appropriately distinguished from one another.

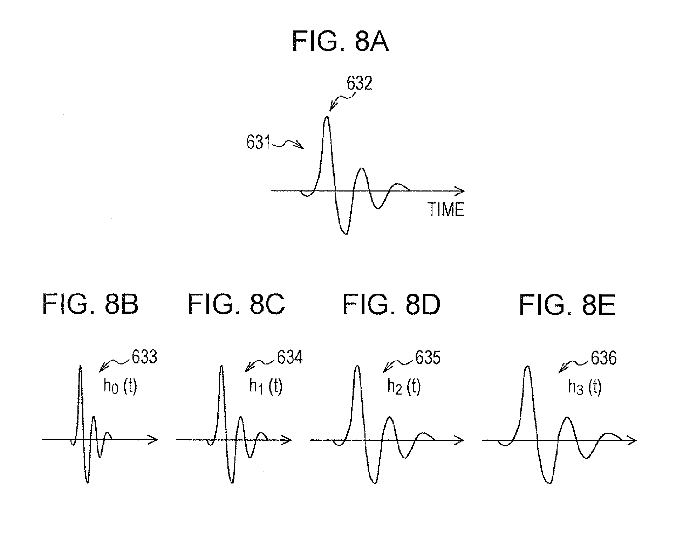

Further, if pulse-based noise is caused mainly by heartbeats, detection accuracy can be improved by using the following feature values in addition to the feature values described above. First, a typical heartbeat signal waveform 631 illustrated in Fig. 8A is prepared in advance. The typical heartbeat signal waveform 631 can be obtained by, for example, picking up multiple heartbeat signals and calculating the average value of the heartbeat signals. When pulse-based noise is detected, the position of the local maximum value 604 in the candidate pulse-based component period and the position of a maximum value 632 of the typical heartbeat signal waveform 631 illustrated in Fig. 8A are aligned with each other. Then, as illustrated in Figs. 8B to 8E, the typical heartbeat signal waveform 631 is expanded and compressed into several scales (633, 634, 635, 636), and the correlation between the heartbeat signal and the input signal is taken at each scale to generate a correlation coefficient. The generated correlation coefficients are added as feature values to the above feature vectors x. If the input signal is a function f(t) at time t and the end points of the candidate pulse-based component period are represented by $t_s$ and $t_e$, a correlation coefficient $r_k$ is calculated using the following formula:

$$rk = \frac{\sum_{t=t_s}^{t_e} h_k(t)f(t)}{\sqrt{\sum_{t=ts}^{t_e} h_k^2(t)\sum_{t=t_s}^{t_e} f^2(t)}}, \qquad k = 0,1,2,3$$

(2)

Functions of Pulse-Based Component Removal Unit

The pulse-based component removal unit 140 includes a signal reduction unit 141, a waveform synthesis unit 142, and a waveform addition unit 143. The signal reduction unit 141 is configured to reduce, in the measured respiratory signal, the signal of the pulse-based component in the detection period detected by the pulse-based component detection unit 130. The waveform synthesis unit 142 is configured to generate a composite waveform from signals near the detection period. The waveform addition unit 143 is configured to add the composite waveform generated by the waveform synthesis unit 142 to the measured respiratory signal.

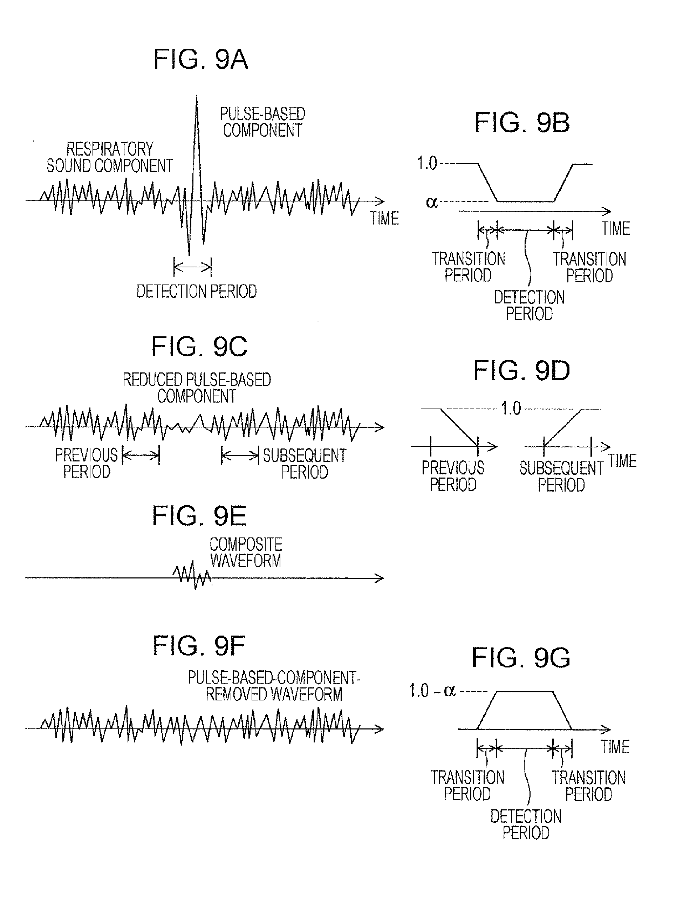

When reducing the signal of the pulse-based component in the detection period illustrated in Fig. 9A, as illustrated in Fig. 9B, the signal reduction unit 141 multiplies the detection period and transition periods provided on both sides with respect to the detection period by weighting functions for reduction. In Fig. 9B, $\alpha$ is a constant that is typically as small as about 0.0 to 0.1. Thus, as illustrated in Fig. 9C, a waveform in which the pulse-based component in the detection period has been reduced can be obtained.

The waveform synthesis unit 142 generates a composite waveform as illustrated in Fig. 9E in order to compensate for the detection period in the waveform illustrated in Fig. 9C in which the pulse-based component has been reduced. The composite waveform can be obtained by, for example, the following procedure: A signal having the same length as the total length of the detection period and the previous and subsequent transition periods before and after the detection period are extracted from each of a previous period and a subsequent period provided before and after the detection period, respectively. After that, each of the previous period and the subsequent period is multiplied by a weighting function as illustrated in Fig. 9D, and then the resulting values are added together. That is, the previous period illustrated in Fig. 9C is multiplied by the weighting function corresponding to the previous period illustrated in Fig. 9D, and the subsequent period illustrated in Fig. 9C is multiplied by the weighting function corresponding to the subsequent period illustrated in Fig. 9D. More specifically, an input signal waveform may be processed while being constantly stored in a buffer having an appropriate length, and, when a pulse-based component is detected, signals at the positions corresponding to the previous period and subsequent period may be copied in different buffers. The buffer having an appropriate length, as used herein, is assumed to have a capacity that is logically about several times the maximum length of the pulse-based component, more specifically, a capacity corresponding to about several seconds.

Alternatively, the waveform portions of the previous period and the subsequent period, which are longer than those described above, may be extracted, and phase alignment is performed so that, for example, the cross-correlation values of the previous period and the subsequent period become minimum. Then, the waveform portions of the most phase-aligned periods may be used, and a composite waveform may be generated using the method described above.

The waveform addition unit 143 adds the composite waveform generated by the waveform synthesis unit 142 to the waveform in which the pulse-based component has been reduced to generate a pulse-based-component-removed waveform illustrated in Fig. 9F. The generation of the pulse-based-component-removed waveform may be implemented by, for example, adding the composite waveform after multiplication by weighting functions for addition, as illustrated in Fig. 9G. The weighting functions for addition illustrated in Fig. 9G are functions determined so that the weighting functions for addition and the weighting functions for reduction illustrated in Fig. 9B constantly equal 1 when added together and the weighting functions for addition and the weighting functions for reduction are complementary to each other.

Functions of Time-To-Frequency Conversion Unit

The time-to-frequency conversion unit 150 performs time-to-frequency conversion on the respiratory signal supplied from the pulse-based component removal unit 140 to generate a time-frequency signal X(t, k). Here, a short-time Fourier transform may be used for time-to-frequency conversion. The time-frequency signal X(t, k) is represented by an amplitude component and a phase component.

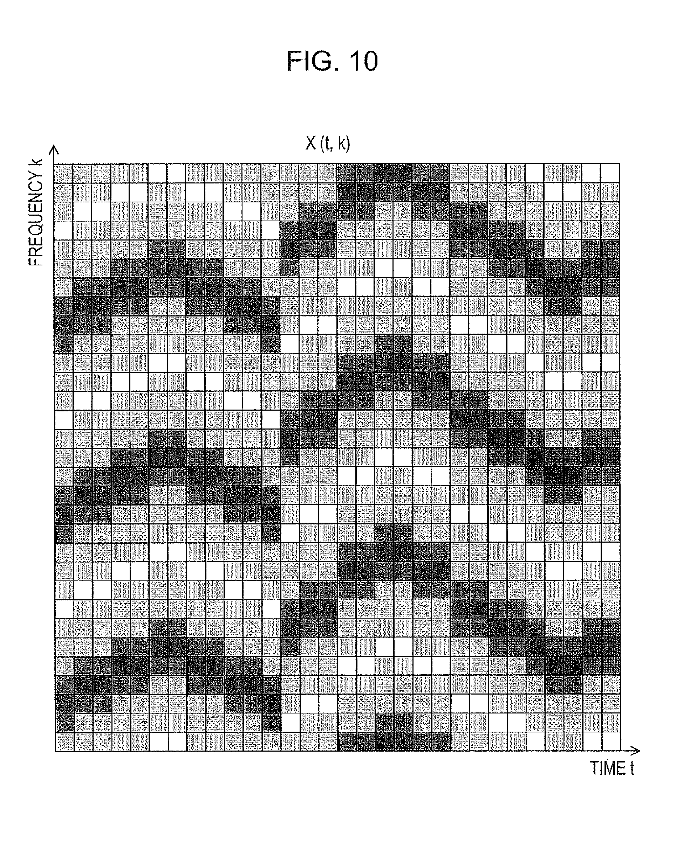

The time-frequency signal obtained by the time-to-frequency conversion unit 150 through time-to-frequency conversion is represented in a manner illustrated in, for example, Fig. 10. A time period t is a discrete time period, and a frequency k is a discrete frequency. An intersection of the time period t and the frequency k represents a time-frequency signal X(t, k). The intensity of the time-frequency signal is represented by gradation, in which dark portions represent high intensity and light portions represent low intensity. That is, assuming a certain time period t, the time-frequency signal shows a certain wavy pattern in accordance with the frequency k. The wavy pattern changes with time.

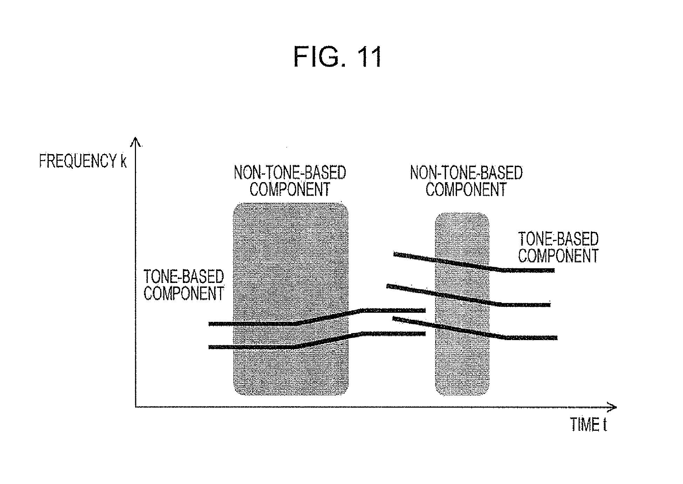

A schematic representation of the time-frequency signal is illustrated in Fig. 11, where it can be found that the tone-based component appears as horizontal linear components. Therefore, the tone-based component can be detected by tracking a local maximum value in the frequency spectrum for each time segment.

Functions of Tone-Based Component Detection Unit

The tone-based component detection unit 160 detects the tone-based component by tracking a peak value (local maximum value) in the time-frequency spectrum for each time segment. The tone-based component detection unit 160 includes a spectral peak detection unit 161, a spectral peak tracking unit 162, and a characteristic-of-tone determination unit 163.

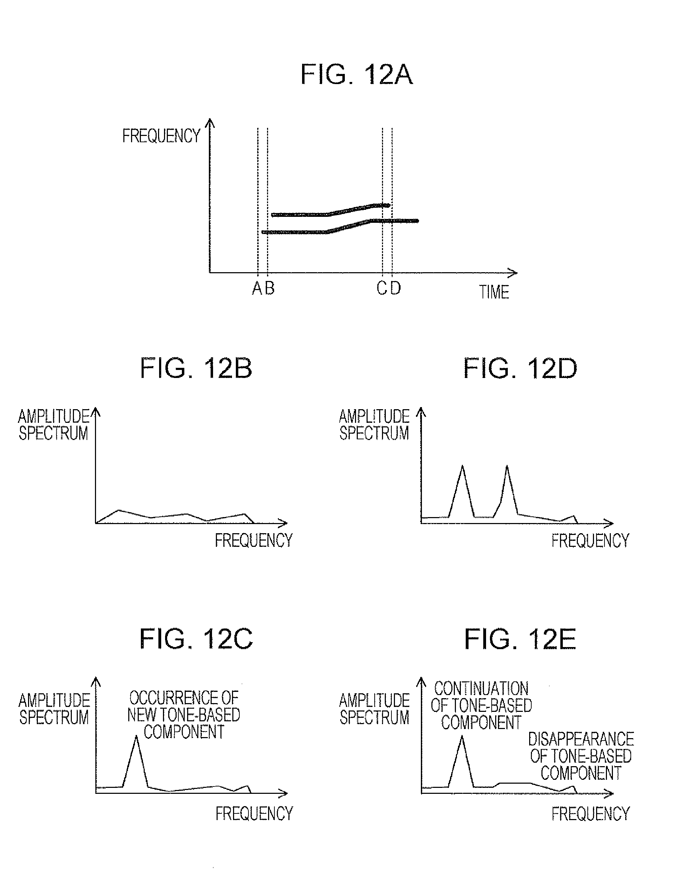

The spectral peak detection unit 161 detects a local maximum value in the time-frequency spectrum for each time segment. For example, in a time-frequency signal as illustrated in Fig. 12A, amplitude spectra at time A, time B, time C, and time D are illustrated in Figs. 12B, 12C, 12D, and 12E, respectively. That is, at the time A, no tone-based component is included and no significant peak is detected, resulting in a gently sloping waveform being obtained. At the time B, one new tone-based component occurs, and the waveform exhibits a local maximum value at one frequency corresponding to the tone-based component. At the time C, another new tone-based component occurs, and the waveform exhibits local maximum values at two frequencies corresponding to the tone-based components. At the time D, one of the tone-based components disappears, and the waveform exhibits a local maximum value at one frequency corresponding to the remaining tone-based component.

The spectral peak tracking unit 162 tracks the local maximum value detected by the spectral peak detection unit 161. When the spectral peak detection unit 161 detects a local maximum value greater than or equal to a certain threshold at time t, the spectral peak tracking unit 162 determines a correspondence between the local maximum value detected at the time t and the local maximum value detected at the preceding time t-1. For example, if the difference between the frequencies falls within a certain range and if the difference in amplitude also falls within a certain range, it is determined that a correspondence exists.

The characteristic-of-tone determination unit 163 determines whether or not the signal components of the local maximum values associated by the spectral peak tracking unit 162 form a tone-based component. That is, if the local maximum values associated by the spectral peak tracking unit 162 are continuous over a period of time greater than or equal to a certain value, it is determined that the signal components form a tone-based component.

Functions of Tone-Based Component Removal Unit

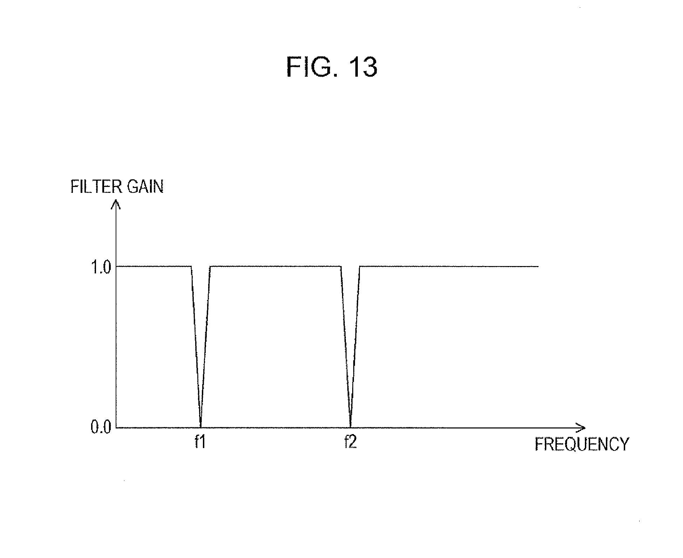

The tone-based component removal unit 170 removes the tone-based component detected by the tone-based component detection unit 160 from the time-frequency spectrum. The detected tone-based component is that as indicated by a thick line in Fig. 12A, and only the corresponding frequency component can be removed or reduced using, for the convenience of ease, a specific-frequency component removal filter as illustrated in Fig. 13 that removes the frequencies of the tone-based component at the respective times.

Functions of Frequency-To-Time Conversion Unit

The frequency-to-time conversion unit 180 performs frequency-to-time conversion on the time-frequency spectrum in which the tone-based component has been removed by the tone-based component removal unit 170. The frequency-to-time conversion can be implemented using, for example, an inverse short-time Fourier transform. Therefore, a signal in which the tone-based component has been removed from the initial measured respiratory signal can be obtained.

Operation of Noise Reducer

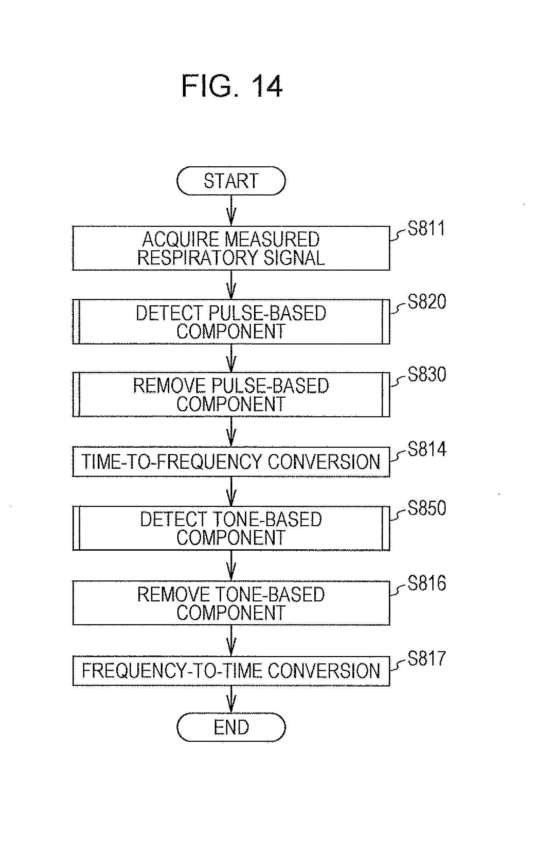

Fig. 14 is a flowchart illustrating an example of a flow of the overall process of the noise reducer 20 according to the first embodiment of the present disclosure. First, a measured respiratory signal measured by the respiratory sound measuring device 10 is acquired (step S811). Then, the pulse-based component detection unit 130 detects a pulse-based component (step S820), and the pulse-based component removal unit 140 removes the detected pulse-based component (step S830),

Further, the time-to-frequency conversion unit 150 performs time-to-frequency conversion on a signal in which the pulse-based component has been removed, and obtains the time-frequency spectrum (step S814). The tone-based component detection unit 160 detects a tone-based component in the time-frequency spectrum (step S850), and the tone-based component removal unit 170 removes the detected tone-based component (step S816). After that, the frequency-to-time conversion unit 180 performs frequency-to-time conversion (step S817), and obtains a signal in which the tone-based component has been removed from the initial measured respiratory signal.

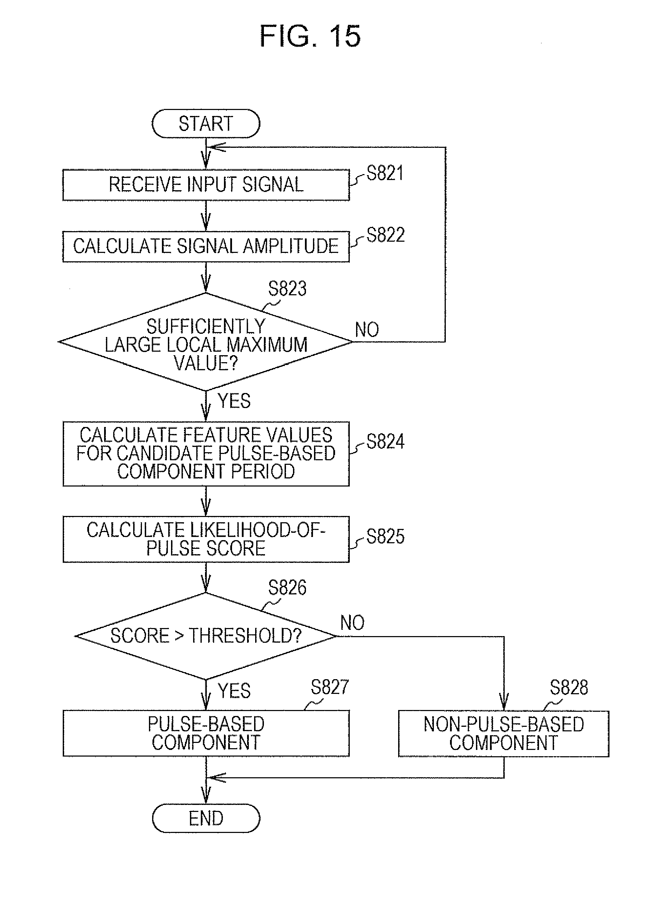

Fig. 15 is a flowchart illustrating an example of a flow of a pulse-based component detection process according to the first embodiment of the present disclosure (step S820). When a measured respiratory signal is input (step S821), the candidate pulse-based component detection unit 131 calculates the amplitude of the measured respiratory signal (step S822), and repeatedly performs this operation until a sufficiently large local maximum value has been obtained (step S823), If a sufficiently large local maximum value is obtained, the candidate pulse-based component detection unit 131 detects, as a candidate pulse-based component period, a period near the local maximum value in which calculated values exceed a certain threshold.

The characteristic-of-pulse score calculation unit 132 calculates feature values for the candidate pulse-based component period (step S824). Then, the calculated feature values are integrated to compute a likelihood-of-pulse score (step S825).

If the likelihood-of-pulse score exceeds a certain threshold (step S826), the characteristic-of-pulse determination unit 133 determines that the subject component is a pulse-based component (step S827), and uses the candidate pulse-based component period as a detection period. Otherwise, it is determined that the subject component is non-pulse-based component (step S828).

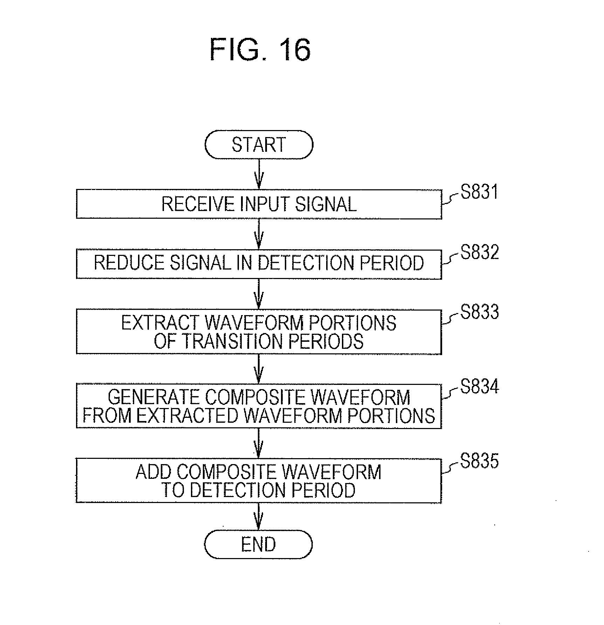

Fig. 16 is a flowchart illustrating an example of a flow of a pulse-based component removal process according to the first embodiment of the present disclosure (step S830). When a measured respiratory signal is input and a pulse-based component is detected (step S831), the signal reduction unit 141 reduces the signal in the detection period (step S832).

The waveform synthesis unit 142 extracts the waveform portions of a previous period and a subsequent period before and after the detection period, respectively, in order to compensate for the detection period in the waveform in which the pulse-based component has been reduced (step S833), and generates a composite waveform (step S834).

Then, the waveform addition unit 143 adds the generated composite waveform to the waveform in which the pulse-based component has been reduced (step S835) to generate a pulse-based-component-removed waveform.

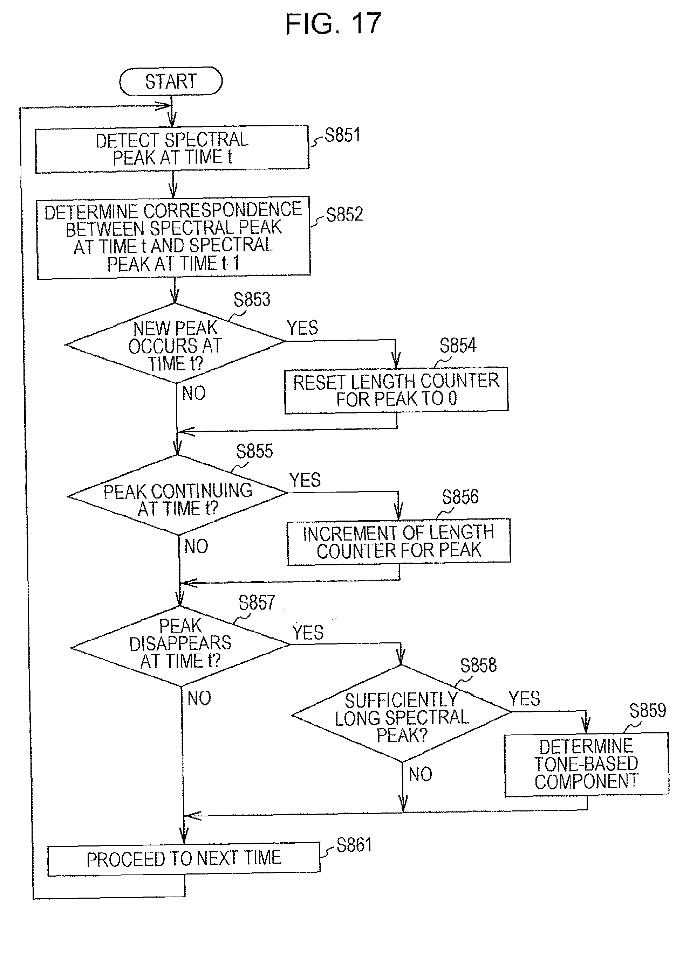

Fig. 17 is a flowchart illustrating an example of a flow of a tone-based component detection process according to the first embodiment of the present disclosure (step S850). When the spectral peak detection unit 161 detects a peak in the frequency domain of the time-frequency spectrum at time t (step S851), the spectral peak tracking unit 162 determines a correspondence between the peak detected at the time t and the peak detected at the preceding time t-1 (step S852).

If a new peak occurs at the time t (step S853), a length counter for the peak is prepared, and the length counter is reset to zero (step S854). If a peak continues at the time t (step S855), the length counter for the peak is incremented by one (step S856).

If a peak disappears at the time t (step S857), when the value of the length counter for the peak is greater than or equal to a certain threshold (step S858), the characteristic-of-tone determination unit 163 determines that the subject component is a tone-based component (step S859), If the value of the length counter for the peak is less than the certain threshold (step S858), the characteristic-of-tone determination unit 163 determines that the subject component is not a tone-based component.

The above series of operations are continuously performed (step S861) to detect a tone-based component having a significant length.

According to the first embodiment of the present disclosure, therefore, a respiratory signal in which the pulse-based component and the tone-based component included in a measured respiratory signal have been removed so that noise can be reduced can be supplied, and respiratory conditions can be easily analyzed from the respiratory signal.

In this first embodiment, both the pulse-based component and the tone-based component are removed; however, one of the pulse-based component and the tone-based component may be removed as necessary. That is, the pulse-based component detection unit 130 and the pulse-based component removal unit 140 may be omitted and only the tone-based component may be removed. Alternatively, the time-to-frequency conversion unit 150, the tone-based component detection unit 160, the tone-based component removal unit 170, and the frequency-to-time conversion unit 180 may be omitted, and only the pulse-based component may be removed.

2. Second Embodiment

Configuration of Respiratory Condition Display System

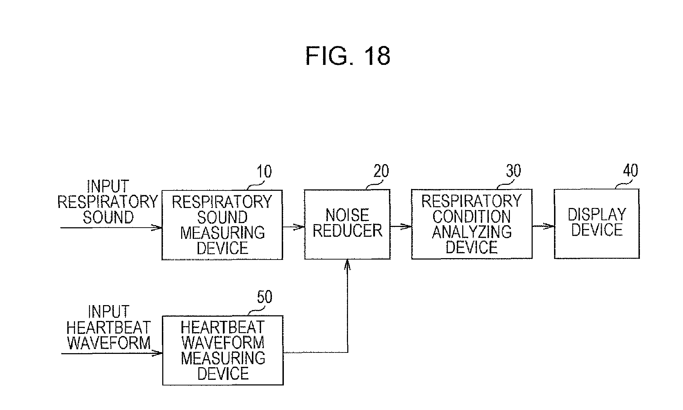

Fig. 18 is a diagram illustrating an example configuration of a respiratory condition display system according to a second embodiment of the present disclosure. The respiratory condition display system according to the second embodiment includes a respiratory sound measuring device 10, a noise reducer 20, a respiratory condition analyzing device 30, a display device 40, and a heartbeat waveform measuring device 50. That is, the respiratory condition display system according to the second embodiment is configured such that the heartbeat waveform measuring device 50 is added to the respiratory condition display system according to the first embodiment. The respiratory sound measuring device 10, the respiratory condition analyzing device 30, and the display device 40 are similar to those in the first embodiment, and descriptions thereof are thus omitted.

The heartbeat waveform measuring device 50 is a measuring device that measures the heartbeat waveform of a living being. The heartbeat waveform measuring device 50 can be implemented as, for example, a pulse oximeter, an electrocardiograph, an acceleration sensor configured to pick up heart sounds, or the like.

The noise reducer 20 according to the second embodiment estimates the heart sound included in the measured respiratory signal on the basis of a measured heartbeat signal that is a heartbeat waveform measured by the heartbeat waveform measuring device 50, and removes or reduces the heart sound. The measured respiratory signal and the measured heartbeat signal are acquired from the same living being, and the heart sound estimated from the measured heartbeat signal is subtracted from the measured respiratory signal to remove or reduce the heart sound as noise of pulse-based component.

Configuration of Noise Reducer

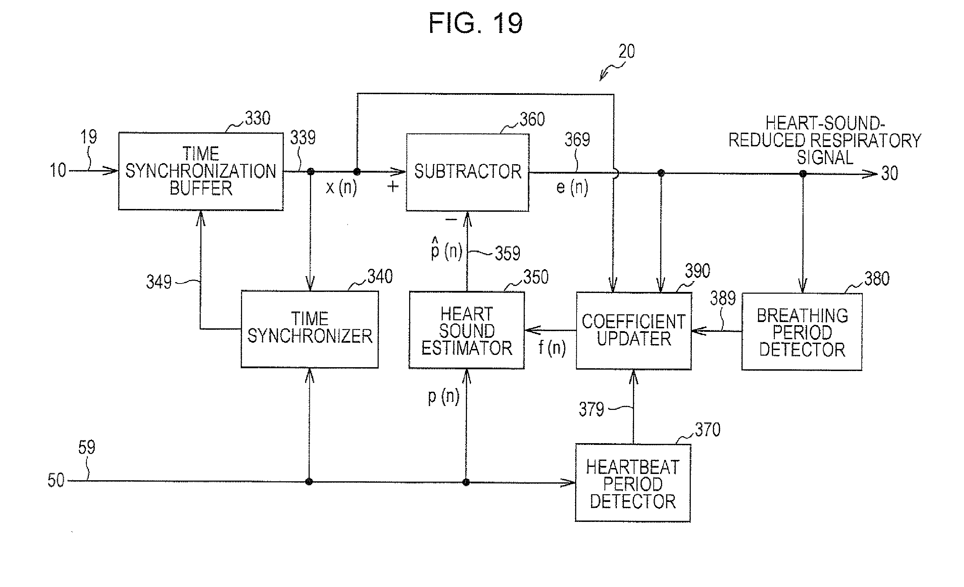

Fig. 19 is a diagram illustrating an example configuration of the noise reducer 20 according to the second embodiment of the present disclosure. The noise reducer 20 includes a time synchronization buffer 330, a time synchronizer 340, a heart sound estimator 350, a subtractor 360, a heartbeat period detector 370, a breathing period detector 380, and a coefficient updater 390. A measured respiratory signal from the respiratory sound measuring device 10 is input to a signal line 19. A measured heartbeat signal from the heartbeat waveform measuring device 50 is input to a signal line 59.

The time synchronizer 340 is configured to synchronize the measured respiratory signal and the measured heartbeat signal with each other by measuring the amount of deviation between the measured respiratory signal and the measured heartbeat signal. The time synchronization buffer 330 is a buffer used to synchronize the measured respiratory signal and the measured heartbeat signal with each other on the basis of the amount of deviation measured by the time synchronizer 340. The respiratory signal synchronized by the time synchronization buffer 330 is supplied to the subtractor 360 via a signal line 339. The time synchronization buffer 330 and the time synchronizer 340 are examples of a time synchronization unit in the appended claims.

The heart sound estimator 350 is configured to estimate the heart sound included in the measured respiratory signal on the basis of the measured heartbeat signal. That is, the heart sound estimator 350 serves a filter that changes a heartbeat waveform obtained as an electrical waveform to a sound-like form. The heart sound estimated by the heart sound estimator 350 is supplied to the subtractor 360 via a signal line 359. The heart sound estimator 350 can be implemented by, for example, a finite impulse response (FIR) filter. The FIR filter performs total sum calculation for tap number k using the following formula:

$$\hat{p}(n) = \sum_{k=0}^{K-1} f(k) p(n-k)$$

(3)

where n denotes the number of samples, p(n) denotes the measured heartbeat signal, p^(n) denotes the estimated heart sound, f(n) denotes the filter coefficient, and K denotes the tap length of the filter coefficient. The tap number k indicates an integer value from 0 to K-1.

The subtractor 360 is configured to subtract the estimated heart sound from the measured respiratory signal. Here, if the measured respiratory signal supplied from the time synchronization buffer 330 is represented by x(n) and the estimated heart sound estimated by the heart sound estimator 350 is represented by p^(n), a heartbeat-sound-reduced respiratory signal e(n) to be output from the subtractor 360 is represented by the following formula:

$$e(n) = x(n) - \hat{p}(n)$$

$$(4)$$

The heartbeat period detector 370 is configured to detect a heartbeat period, which is a period including the heart sound in the measured respiratory signal, on the basis of the measured heartbeat signal. Information indicating whether or not the subject period is a heartbeat period is supplied to the coefficient updater 390 via a signal line 379. The breathing period detector 380 is configured to detect a breathing period, which is a period including the respiratory sound in the measured respiratory signal, on the basis of the heartbeat-sound-reduced respiratory signal output from the subtractor 360. Information indicating whether or not the subject period is a breathing period is supplied to the coefficient updater 390 via a signal line 389. The heartbeat period detector 370 is an example of a heartbeat period detection unit in the appended claims. The breathing period detector 380 is an example of a breathing period detection unit in the appended claims.

The coefficient updater 390 is configured to update the filter coefficient of the heart sound estimator 350. The filter coefficient is updated so that a mean square error between the estimated heart sound p^(n) that is the filter output and the measured respiratory signal x(n) becomes minimum. That is, a mean square error of the difference e(n) between the estimated heart sound p^(n) and the measured respiratory signal x(n) is used as an evaluation function J, which is defined by the following formula:

$$J = \{x(n) - \hat{p}(n)\}^2 = e^2(n)$$

$$(5)$$

Substituting formula (5) into formula (3) above and taking partial derivative with f as a constant yield a formula for updating the coefficient f as follows:

$$f(n+1) = f(n) + \mu e(n)x(n - k),$$

where $\mu$ denotes the convergence constant and the constant may be determined empirically or may be changed in accordance with the state of the input signal using the learning identification method. In the above formula, furthermore, x(n - k) denotes the heart sound without respiratory sounds. A heart sound signal including no respiratory sounds can be picked up by causing a living being to consciously stop breathing for about several seconds, and can be used as an initial value to generate a filter coefficient.

The coefficient updater 390 updates the filter coefficient using a signal that is in a non-breathing period and in a heartbeat period. Thus, the coefficient updater 390 receives information from the heartbeat period detector 370 and the breathing period detector 380, and updates the filter coefficient at the timing of a non-breathing period and of a heartbeat period. Therefore, the coefficient can be updated using a signal portion where respiratory sounds and heart sounds do not overlap, and the heart sound estimator 350 makes feasible heart sound estimation with less noise. The coefficient updater 390 is an example of a coefficient update unit in the appended claims.

Overview of Process of Noise Reducer

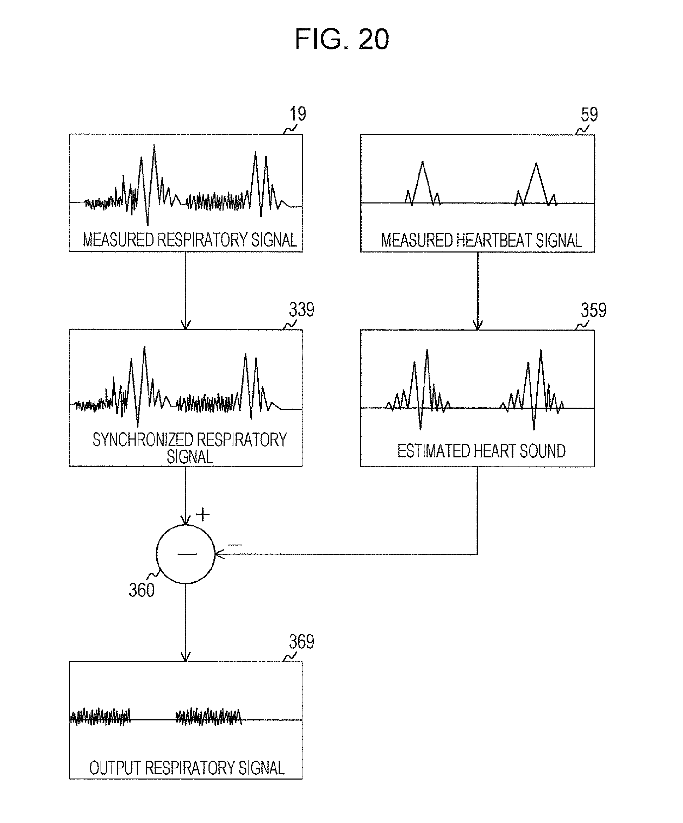

Fig. 20 is a diagram illustrating an overview of the process of the noise reducer 20 according to the second embodiment of the present disclosure.
The heart sound estimator 350 estimates heart sound in the measured heartbeat signal input from the heartbeat waveform measuring device 50 via the signal line 59 before supplying the measured heartbeat signal to the subtractor 360 via the signal line 359. The measured respiratory signal input from the respiratory sound measuring device 10 via the signal line 19 is subjected to synchronization by the time synchronization buffer 330 and the time synchronizer 340 and is supplied to the subtractor 360 via the signal line 339. The subtractor 360 subtracts the estimated heart sound from the synchronized respiratory signal, and outputs the respiratory signal in which the heart sound has been reduced.

Configuration of Time Synchronizer

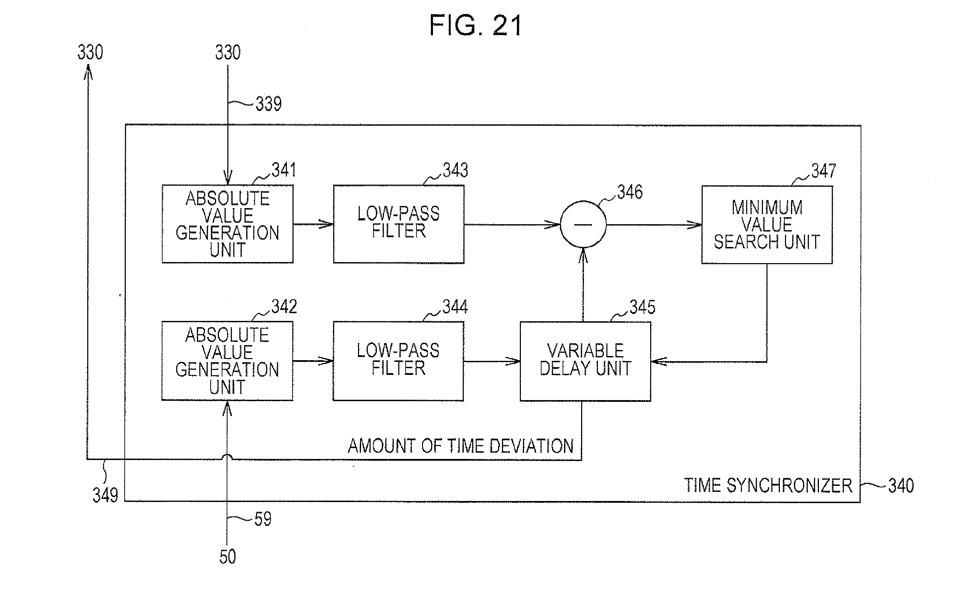

Fig. 21 is a diagram illustrating an example configuration of the time synchronizer 340 according to the second embodiment of the present disclosure. The time synchronizer 340 includes absolute value generation units 341 and 342, low-pass filters 343 and 344, a variable delay unit 345, a subtractor 346, and a minimum value search unit 347.
The absolute value generation unit 341 is configured to generate the absolute value of the measured respiratory signal supplied from the time synchronization buffer 330 via the signal line 339. The low-pass filter 343 is a filter that allows only the low-frequency components of the output of the absolute value generation unit 341 to pass therethrough. Therefore, a waveform in which the high-frequency components have been removed from the measured respiratory signal can be obtained.
The absolute value generation unit 342 is configured to generate the absolute value of the measured heartbeat signal input from the heartbeat waveform measuring device 50 via the signal line 59. The low-pass filter 344 is a filter that allows only the low-frequency components of the output of the absolute value generation unit 342 to pass therethrough. Therefore, a waveform in which the high-frequency components have been removed from the measured heartbeat signal can be obtained.
The variable delay unit 345 is configured to delay the output of the low-pass filter 344 in accordance with an instruction from the minimum value search unit 347. The variable delay unit 345 further supplies the amount of time deviation corresponding to the time by which the output of the low-pass filter 344 is delayed to the time synchronization buffer 330 via a signal line 349.
The subtractor 346 is configured to generate the difference between the output of the low-pass filter 343 and the output of the low-pass filter 344 that has been delayed by the variable delay unit 345.
The minimum value search unit 347 is configured to search for an amount of time deviation for which the difference generated by the subtractor 346 becomes a minimum value. That is, the minimum value search unit 347 sequentially changes the delay time to be supplied to the variable delay unit 345, and, when the difference generated by the subtractor 346 becomes a minimum value, the current delay time is output from the variable delay unit 345 as the amount of time deviation.

Configuration of Time Synchronization Buffer

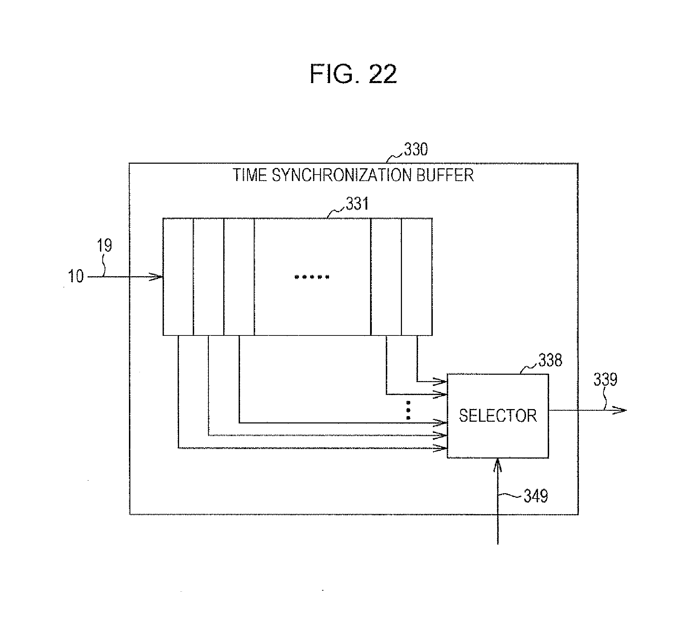

Fig. 22 is a diagram illustrating an example configuration of the time synchronization buffer 330 according to the second embodiment of the present disclosure. The time synchronization buffer 330 includes a multiple-stage FIFO buffer 331 and a selector 338.
The FIFO buffer 331 is a first-in first-out (FIFO) buffer that sequentially holds a measured respiratory signal input from the respiratory sound measuring device 10 via the signal line 19. A measured respiratory signal is input to the FIFO buffer 331 at certain time intervals, and the stages of the FIFO buffer 331 are shifted one-by-one each time a new measured respiratory signal is input. Therefore, each stage of the FIFO buffer 331 holds a measured respiratory signal having a delay that is a constant multiple of the certain time interval. The FIFO buffer 331 is configured such that a value can be read from each stage, and the read values are supplied to the selector 338.
The selector 338 is configured to select one of the values held in the respective stages of the FIFO buffer 331. The selector 338 is supplied with the amount of time deviation from the time synchronizer 340 via the signal line 349, and a value held in the stage of the FIFO buffer 331, which corresponds to the amount of time deviation, is selected and is output to the signal line 339. That is, a measured respiratory signal having a delay of the amount of time deviation supplied from the time synchronizer 340 is output via the signal line 339.

Configuration of Heartbeat Period Detector

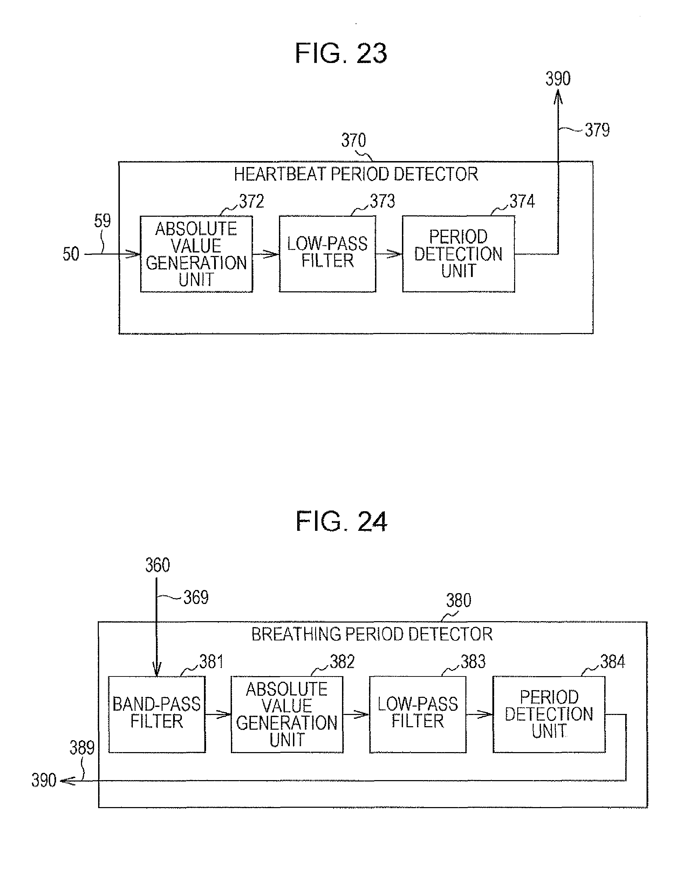

Fig. 23 is a diagram illustrating an example configuration of the heartbeat period detector 370 according to the second embodiment of the present disclosure. The heartbeat period detector 370 includes an absolute value generation unit 372, a low-pass filter 373, and a period detection unit 374.

The absolute value generation unit 372 is configured to generate the absolute value of the measured heartbeat signal input from the heartbeat waveform measuring device 50 via the signal line 59. The low-pass filter 373 is a filter that allows only the low-frequency components of the output of the absolute value generation unit 372 to pass therethrough. Therefore, a waveform in which the high-frequency components have been removed from the measured heartbeat signal can be obtained.

The period detection unit 374 is configured to detect a period in the heartbeat signal output from the low-pass filter 373 in which values exceed a certain threshold. That is, the period detection unit 374 detects, as a heartbeat period, a period from the time when values exceed the certain threshold to the time when the values are below the certain threshold again. Here, the certain threshold may be, for example, about 10 percent of an average peak value. Information indicating whether or not the current period is the heartbeat period detected by the period detection unit 374 is supplied to the coefficient updater 390 via the signal line 379.

Configuration of Breathing Period Detector

Fig. 24 is a diagram illustrating an example configuration of the breathing period detector 380 according to the second embodiment of the present disclosure. The breathing period detector 380 includes a band-pass filter 381, an absolute value generation unit 382, a low-pass filter 383, and a period detection unit 384.

The band-pass filter 381 is a filter that allows only the signal components in a certain frequency band in the heartbeat-sound-reduced respiratory signal output from the subtractor 360 via the signal line 369 to pass therethrough. Here, since it is taken into account that respiratory sounds are in a band of up to about 2 KHz and heart sounds are in a band less than or equal to 100 Hz, the certain frequency band may be a frequency band of, for example, 100 Hz to 2 KHz.

The absolute value generation unit 382 is configured to generate the absolute value of the signal transmitted through the band-pass filter 381. The low-pass filter 383 is a filter that allows only the low-frequency components of the output of the absolute value generation unit 382 to pass therethrough. The frequency at which signals are transmitted through the low-pass filter 383 may be, for example, about 5 Hz in consideration of the frequency of breaths a living being takes. Therefore, a waveform in which the component necessary for detecting a breathing period has been extracted from the heartbeat-sound-reduced respiratory signal can be obtained.

The period detection unit 384 is configured to detect a period in the respiratory signal output from the low-pass filter 383 in which values exceed a certain threshold. That is, the period detection unit 384 detects, as a heartbeat period, a period from the time when values exceed the certain threshold to the time when the values are below the certain threshold again. Here, the certain threshold may be, for example, about 10 percent of an average peak value. Information indicating whether or not the current period is a breathing period, which has been detected by the period detection unit 384, is supplied to the coefficient updater 390 via the signal line 389.

Operation of Noise Reducer

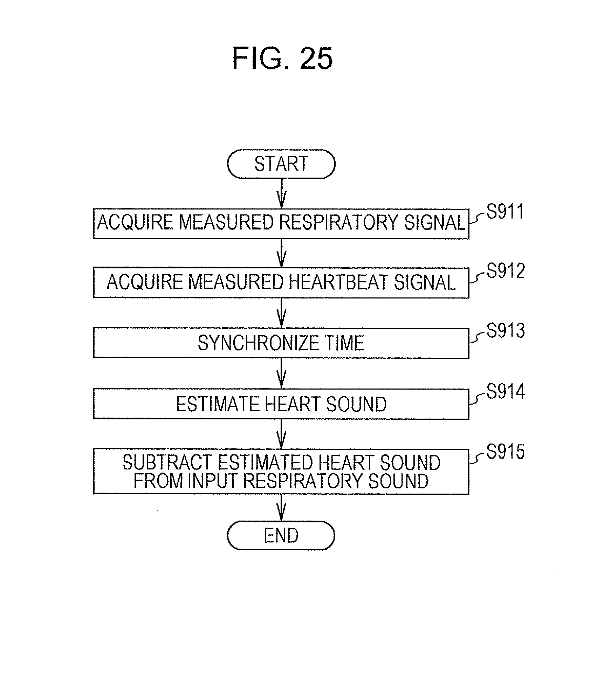

Fig. 25 is a flowchart illustrating an example of a flow of the overall process of the noise reducer 20 according to the second embodiment of the present disclosure. First, a measured respiratory signal measured by the respiratory sound measuring device 10 is acquired (step S911). A measured heartbeat signal measured by the heartbeat waveform measuring device 50 is also acquired (step S912). Then, the measured respiratory signal and the measured heartbeat signal are synchronized in time with each other by the time synchronization buffer 330 and the time synchronizer 340 (step S913).

Further, the heart sound estimator 350 estimates the heart sound included in the measured respiratory signal on the basis of the measured heartbeat signal, and outputs the estimated heart sound (step S914). Then, the subtractor 360 subtracts the estimated heart sound from the measured respiratory signal (step S915). Therefore, a heartbeat-sound-reduced respiratory signal can be obtained.

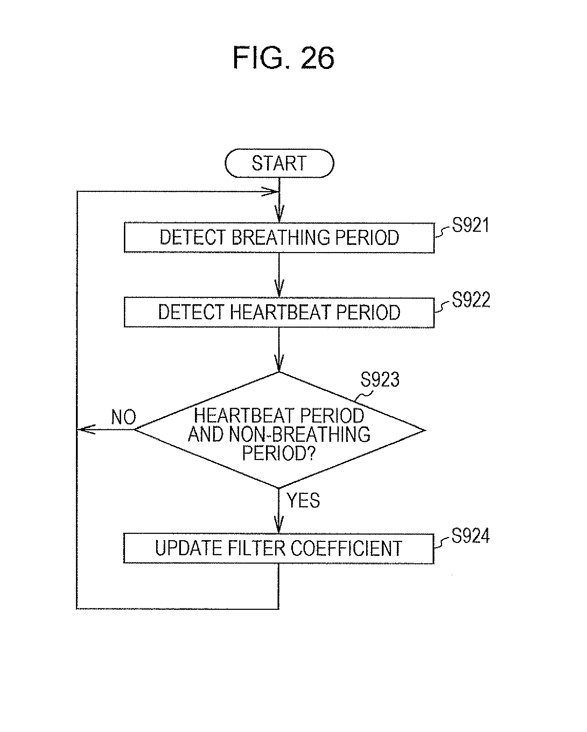

Fig. 26 is a flowchart illustrating an example of a flow of a filter update process according to the second embodiment of the present disclosure. First, the breathing period detector 380 detects a breathing period in the measured respiratory signal on the basis of the heartbeat-sound-reduced respiratory signal (step S921). Further, the heartbeat period detector 370 detects a heartbeat period in the measured respiratory signal on the basis of the measured heartbeat signal (step S922).

Then, if the subject period is a heartbeat period and is a non-breathing period (step S923), the coefficient updater 390 updates the filter coefficient of the heart sound estimator 350 (step S924).

The above filter update process is repeatedly performed constantly in parallel with the overall process of the noise reducer 20. Therefore, heart sounds can be removed in accordance with the conditions of a living being when a respiratory signal is acquired from the living being.

According to the second embodiment of the present disclosure, therefore, a respiratory signal in which the heart sound included in a measured respiratory signal has been removed so that noise can be reduced can be supplied, and respiratory conditions can be easily analyzed from the respiratory signal.

The embodiments of the present disclosure are illustrated by way of example to realize the present disclosure. As clearly illustrated in the embodiments of the present disclosure, there is a correspondence between the features of the embodiments of the present disclosure and the features of the appended claims. There is also a correspondence between the elements in the appended claims and the elements in the embodiments of the present disclosure which have the same names as those of the elements in the appended claims. However, the present disclosure is not limited to the embodiments, and a variety of modifications can be made to the embodiments without departing from the scope of the present disclosure to realize the present disclosure.

Additionally, the process procedures described in the embodiments of the present disclosure may be regarded as methods having the above series of procedures, or may be regarded as programs which enable a computer to execute the above series of procedures or may be regarded recording media storing the programs. Examples of the recording media may include a compact disc (CD), a MiniDisc (MD), a digital versatile disk (DVD), a memory card, and a Blu-ray Disc (registered trademark).

The present disclosure contains subject matter related to that disclosed in Japanese Priority Patent Application JP 2010-278706 filed in the Japan Patent Office on December 15, 2010.

It should be understood by those skilled in the art that various modifications, combinations, sub-combinations and alterations may occur depending on design requirements and other factors insofar as they are within the scope of the appended claims.

In so far as the embodiments of the invention described above are implemented, at least in part, using software-controlled data processing apparatus, it will be appreciated that a computer program providing such software control and a transmission, storage or other medium by which such a computer program is provided are envisaged as aspects of the present invention.

**Claims**

1. A respiratory signal processing apparatus comprising:

   a pulse-based component detection unit configured to detect a pulse-based component from a first signal acquired from a living being; and
   a pulse-based component removal unit configured to remove the detected pulse-based component from a second signal acquired from the living being, the second signal including respiratory sounds.

2. The respiratory signal processing apparatus according to Claim 1, wherein the first signal and the second signal are measured respiratory signals obtained by measuring same respiratory sounds, and
   wherein the pulse-based component detection unit includes
   a candidate pulse-based component detection unit configured to detect, as a candidate pulse-based component period, a period including a local maximum value in the first signal,
   a score calculation unit configured to calculate a likelihood-of-pulse score for the candidate pulse-based component period, and
   a characteristic-of-pulse determination unit configured to determine that a signal included in a detection period is the pulse-based component, the detection period being a candidate pulse-based component period for which the likelihood-of-pulse score satisfies a certain threshold.

3. The respiratory signal processing apparatus according to Claim 2, wherein the pulse-based component removal unit includes
   a signal reduction unit configured to reduce the detected pulse-based component in the first signal,
   a waveform synthesis unit configured to generate a composite waveform from a signal near the detection period, and
   a waveform addition unit configured to add the composite waveform to the first signal.

4. The respiratory signal processing apparatus according to Claim 1, further comprising a time synchronization unit,
   wherein the first signal is a measured heartbeat signal obtained by measuring a heartbeat waveform of the living being,
   wherein the second signal is a measured respiratory signal obtained by measuring respiratory sounds of the living being,
wherein the time synchronization unit is configured to synchronize the measured respiratory signal and the measured heartbeat signal with each other,
wherein the pulse-based component detection unit detects the pulse-based component by estimating heart sounds from the measured heartbeat signal, and
wherein the pulse-based component removal unit removes the pulse-based component by removing the estimated heart sounds from the synchronized measured respiratory signal.

5. The respiratory signal processing apparatus according to Claim 4, further comprising:

a breathing period detection unit configured to detect a breathing period on the basis of a measured respiratory signal in which the heart sounds have been removed;
a heartbeat period detection unit configured to detect a heartbeat period on the basis of the measured heartbeat signal; and
a coefficient update unit configured to update a filter coefficient,

wherein the pulse-based component detection unit is a finite impulse response filter, and wherein the coefficient update unit updates a filter coefficient of the finite impulse response filter during a period that is not the breathing period but is the heartbeat period.

6. The respiratory signal processing apparatus according to Claim 1, further comprising:

a time-to-frequency conversion unit configured to convert the second signal in which the pulse-based component has been removed to generate a frequency spectrum signal for each time segment;
a tone-based component detection unit configured to detect a tone-based component from the frequency spectrum signal;
a tone-based component removal unit configured to remove the detected tone-based component from the frequency spectrum signal; and
a frequency-to-time conversion unit configured to inversely convert the frequency spectrum signal in which the tone-based component has been removed.

7. The respiratory signal processing apparatus according to Claim 6, wherein the tone-based component detection unit includes
a spectral peak detection unit configured to detect a peak in the frequency spectrum signal for each time segment,
a spectral peak tracking unit configured to track over time segments a peak in the frequency spectrum signal for each time segment, and
a characteristic-of-tone determination unit configured to determine whether or not the tone-based component exists in accordance with a peak tracked by the spectral peak tracking unit.

8. A respiratory signal processing apparatus comprising:

a time-to-frequency conversion unit configured to convert a measured respiratory signal obtained by measuring respiratory sounds of a living being to generate a frequency spectrum signal for each time segment;
a tone-based component detection unit configured to detect a tone-based component from the frequency spectrum signal;
a tone-based component removal unit configured to remove the detected tone-based component from the frequency spectrum signal; and
a frequency-to-time conversion unit configured to inversely convert the frequency spectrum signal in which the tone-based component has been removed.

9. A respiratory signal processing method comprising:

detecting, as a candidate pulse-based component period, a period including a local maximum value in a measured respiratory signal obtained by measuring respiratory sounds of a living being;
calculating a likelihood-of-pulse score for the candidate pulse-based component period;
determining, as the pulse-based component, a signal included in a detection period, the detection period being a candidate pulse-based component period for which the likelihood-of-pulse score satisfies a certain threshold;
reducing the detected pulse-based component in the measured respiratory signal;

generating a composite waveform from a signal near the detection period; and
adding the composite waveform to the measured respiratory signal to remove the detected pulse-based component.

**10.** A respiratory signal processing method comprising:

converting a measured respiratory signal obtained by measuring respiratory sounds of a living being to generate a frequency spectrum signal for each time segment;
detecting a tone-based component from the frequency spectrum signal;
removing the detected tone-based component from the frequency spectrum signal; and
performing frequency-to-time conversion by inversely converting the frequency spectrum signal in which the tone-based component has been removed.

**11.** A respiratory signal processing method comprising:

synchronizing a measured respiratory signal and a measured heartbeat signal with each other, the measured respiratory signal being obtained by measuring respiratory sounds of a living being, the measured heartbeat signal being obtained by measuring a heartbeat waveform of the living being;
estimating heart sounds from the measured heartbeat signal; and
removing the estimated heart sounds from the synchronized measured respiratory signal.

**12.** A program for causing a computer to execute:

detecting, as a candidate pulse-based component period, a period including a local maximum value in a measured respiratory signal obtained by measuring respiratory sounds of a living being;
calculating a likelihood-of-pulse score for the candidate pulse-based component period;
determining, as the pulse-based component, a signal included in a detection period, the detection period being a candidate pulse-based component period for which the likelihood-of-pulse score satisfies a certain threshold;
reducing the detected pulse-based component in the measured respiratory signal;
generating a composite waveform from a signal near the detection period; and
adding the composite waveform to the measured respiratory signal to remove the detected pulse-based component.

**13.** A program for causing a computer to execute:

converting a measured respiratory signal obtained by measuring respiratory sounds of a living being to generate a frequency spectrum signal for each time segment;
detecting a tone-based component from the frequency spectrum signal;
removing the detected tone-based component from the frequency spectrum signal; and
performing frequency-to-time conversion by inversely converting the frequency spectrum signal in which the tone-based component has been removed.

**14.** A program for causing a computer to execute:

synchronizing a measured respiratory signal and a measured heartbeat signal with each other, the measured respiratory signal being obtained by measuring respiratory sounds of a living being, the measured heartbeat signal being obtained by measuring a heartbeat waveform of the living being;
estimating heart sounds from the measured heartbeat signal; and
removing the estimated heart sounds from the synchronized measured respiratory signal.

FIG. 1

INPUT
RESPIRATORY
SOUND

10

RESPIRATORY
SOUND
MEASURING
DEVICE

20

NOISE
REDUCER

30

RESPIRATORY
CONDITION
ANALYZING
DEVICE

40

DISPLAY
DEVICE

FIG. 2A

BREATHING PERIOD

HEART THROB

FIG. 2B

BREATHING PERIOD

2000Hz
1000Hz

100Hz

HEART THROB

EP 2 465 437 A2

# FIG. 3A

# FIG. 3B

# FIG. 4

FIG. 5A

FIG. 5B

## FIG. 6

FIG. 7A

601 602

TIME

FIG. 7B

603 604

TIME

FIG. 7C

605

TIME

606

FIG. 7D

607

611 612

TIME

613

608

FIG. 7E

620

POWER

$X_5$ $X_6$ $X_7$ $X_8$

FREQUENCY

# FIG. 8A

# FIG. 8B   FIG. 8C   FIG. 8D   FIG. 8E

# FIG. 9A

PULSE-BASED
COMPONENT

RESPIRATORY
SOUND COMPONENT

TIME

DETECTION PERIOD

# FIG. 9B

1.0

α

TIME

TRANSITION
PERIOD

TRANSITION
PERIOD

DETECTION
PERIOD

# FIG. 9C

REDUCED PULSE-BASED
COMPONENT

PREVIOUS
PERIOD

SUBSEQUENT
PERIOD

# FIG. 9D

1.0

PREVIOUS
PERIOD

SUBSEQUENT
PERIOD

TIME

# FIG. 9E

COMPOSITE
WAVEFORM

# FIG. 9F

PULSE-BASED-COMPONENT-
REMOVED WAVEFORM

# FIG. 9G

1.0 – α

TIME

TRANSITION
PERIOD

TRANSITION
PERIOD

DETECTION
PERIOD

FIG. 10

X (t, k)

FREQUENCY k

TIME t

# FIG. 11

# FIG. 12A

FREQUENCY

A B    C D    TIME

# FIG. 12B

AMPLITUDE SPECTRUM

FREQUENCY

# FIG. 12D

AMPLITUDE SPECTRUM

FREQUENCY

# FIG. 12C

AMPLITUDE SPECTRUM

OCCURRENCE OF NEW TONE-BASED COMPONENT

FREQUENCY

# FIG. 12E

AMPLITUDE SPECTRUM

CONTINUATION OF TONE-BASED COMPONENT

DISAPPEARANCE OF TONE-BASED COMPONENT

FREQUENCY

FIG. 13

# FIG. 14

START

ACQUIRE MEASURED RESPIRATORY SIGNAL — S811

DETECT PULSE-BASED COMPONENT — S820

REMOVE PULSE-BASED COMPONENT — S830

TIME-TO-FREQUENCY CONVERSION — S814

DETECT TONE-BASED COMPONENT — S850

REMOVE TONE-BASED COMPONENT — S816

FREQUENCY-TO-TIME CONVERSION — S817

END

# FIG. 15

```
                    START

                       ↓
        RECEIVE INPUT SIGNAL              S821

                       ↓
      CALCULATE SIGNAL AMPLITUDE          S822

                       ↓
                                   S823
            SUFFICIENTLY                        NO
         LARGE LOCAL MAXIMUM
              VALUE?

                       ↓ YES

       CALCULATE FEATURE VALUES
      FOR CANDIDATE PULSE-BASED           S824
          COMPONENT PERIOD

                       ↓
       CALCULATE LIKELIHOOD-OF-
             PULSE SCORE                  S825

                       ↓
                             S826
          SCORE > THRESHOLD?                    NO

              ↓ YES        S827                        S828
        PULSE-BASED                       NON-PULSE-BASED
        COMPONENT                            COMPONENT

                       ↓
                     END
```

# FIG. 16

```
                    ┌─────────────┐
                    │    START    │
                    └──────┬──────┘
                           │
                           ▼
        ┌──────────────────────────────────┐  S831
        │       RECEIVE INPUT SIGNAL        │
        └──────────────────┬───────────────┘
                           │
                           ▼
        ┌──────────────────────────────────┐  S832
        │  REDUCE SIGNAL IN DETECTION PERIOD│
        └──────────────────┬───────────────┘
                           │
                           ▼
        ┌──────────────────────────────────┐  S833
        │      EXTRACT WAVEFORM PORTIONS    │
        │        OF TRANSITION PERIODS      │
        └──────────────────┬───────────────┘
                           │
                           ▼
        ┌──────────────────────────────────┐  S834
        │    GENERATE COMPOSITE WAVEFORM    │
        │ FROM EXTRACTED WAVEFORM PORTIONS  │
        └──────────────────┬───────────────┘
                           │
                           ▼
        ┌──────────────────────────────────┐  S835
        │       ADD COMPOSITE WAVEFORM      │
        │         TO DETECTION PERIOD       │
        └──────────────────┬───────────────┘
                           │
                           ▼
                    ┌─────────────┐
                    │     END     │
                    └─────────────┘
```

# FIG. 17

```
                        ┌──────────┐
                        │  START   │
                        └──────────┘
                             │
                             ▼
                 ┌──────────────────────┐  ╭S851
                 │   DETECT SPECTRAL     │
                 │    PEAK AT TIME t     │
                 └──────────────────────┘
                             │
                             ▼
                 ┌──────────────────────┐  ╭S852
                 │ DETERMINE CORRESPONDENCE │
                 │ BETWEEN SPECTRAL PEAK │
                 │ AT TIME t AND SPECTRAL│
                 │   PEAK AT TIME t-1    │
                 └──────────────────────┘
                             │
                          S853│
                             ▼
                        ╱─────────╲        YES
                       ╱ NEW PEAK  ╲─────────────┐        S854
                      ⟨  OCCURS AT  ⟩             ▼
                       ╲  TIME t?  ╱      ┌──────────────────┐
                        ╲─────────╱       │ RESET LENGTH COUNTER │
                            │NO           │   FOR PEAK TO 0   │
                            │             └──────────────────┘
                            │◄───────────────────┘
                          S855│
                             ▼
                        ╱─────────╲        YES
                       ╱   PEAK    ╲─────────────┐        S856
                      ⟨ CONTINUING  ⟩            ▼
                       ╲ AT TIME t? ╱     ┌──────────────────┐
                        ╲─────────╱       │ INCREMENT OF LENGTH │
                            │NO           │  COUNTER FOR PEAK │
                            │             └──────────────────┘
                            │◄───────────────────┘
                          S857│
                             ▼
                        ╱─────────╲        YES
                       ╱   PEAK    ╲──────────────┐
                      ⟨ DISAPPEARS  ⟩             ▼          S858
                       ╲ AT TIME t? ╱        ╱─────────╲         YES
                        ╲─────────╱         ╱ SUFFICIENTLY╲──────────┐   S859
                            │NO            ⟨ LONG SPECTRAL ⟩         ▼
                            │               ╲   PEAK?   ╱    ┌──────────────┐
                            │                ╲─────────╱     │  DETERMINE   │
                            │                    │NO         │  TONE-BASED  │
                            │                    │           │  COMPONENT   │
                            │◄───────────────────┘           └──────────────┘
                            ▼                                        │
                 ┌──────────────────────┐  ╭S861                     │
                 │  PROCEED TO NEXT TIME │◄─────────────────────────┘
                 └──────────────────────┘
                            │
                            └──────────────► (back to START)
```

# FIG. 18

INPUT
RESPIRATORY
SOUND

RESPIRATORY
SOUND
MEASURING
DEVICE
10

NOISE
REDUCER
20

RESPIRATORY
CONDITION
ANALYZING
DEVICE
30

DISPLAY
DEVICE
40

INPUT
HEARTBEAT
WAVEFORM

HEARTBEAT
WAVEFORM
MEASURING
DEVICE
50

# FIG. 19

EP 2 465 437 A2

# FIG. 20

19

MEASURED RESPIRATORY SIGNAL

59

MEASURED HEARTBEAT SIGNAL

339

SYNCHRONIZED RESPIRATORY SIGNAL

359

ESTIMATED HEART SOUND

360

369

OUTPUT RESPIRATORY SIGNAL

# FIG. 21

EP 2 465 437 A2

FIG. 22

TIME SYNCHRONIZATION BUFFER ~330

~331

• • • • •

SELECTOR ~338

339

~349

# FIG. 23

390

~379

370

HEARTBEAT PERIOD DETECTOR

59

50

~372
ABSOLUTE VALUE GENERATION UNIT

~373
LOW-PASS FILTER

~374
PERIOD DETECTION UNIT

# FIG. 24

360

~369

380

BREATHING PERIOD DETECTOR

~381
BAND-PASS FILTER

~382
ABSOLUTE VALUE GENERATION UNIT

~383
LOW-PASS FILTER

~384
PERIOD DETECTION UNIT

389

390

# FIG. 25

```
                    ( START )
                        |
                        v
   ACQUIRE MEASURED RESPIRATORY SIGNAL  --S911
                        |
                        v
   ACQUIRE MEASURED HEARTBEAT SIGNAL  --S912
                        |
                        v
        SYNCHRONIZE TIME  --S913
                        |
                        v
        ESTIMATE HEART SOUND  --S914
                        |
                        v
   SUBTRACT ESTIMATED HEART SOUND  --S915
   FROM INPUT RESPIRATORY SOUND
                        |
                        v
                    ( END )
```

# FIG. 26

```
        ┌─────────┐
        │  START  │
        └────┬────┘
             │
             ▼
   ┌──────────────────────────┐   S921
   │  DETECT BREATHING PERIOD │
   └──────────────┬───────────┘
                  │
                  ▼
   ┌──────────────────────────┐   S922
   │  DETECT HEARTBEAT PERIOD │
   └──────────────┬───────────┘
                  │
                  ▼
              S923
         ◇──────────────◇
   NO   ╱ HEARTBEAT PERIOD ╲
◄──────╱  AND NON-BREATHING ╲
       ╲     PERIOD?        ╱
         ◇──────────────◇
                  │ YES
                  ▼
   ┌──────────────────────────┐   S924
   │  UPDATE FILTER COEFFICIENT │
   └──────────────────────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2628690 B
- JP 2010278706 A